# EUROPEAN PATENT SPECIFICATION

(11) **EP 2 430 442 B1**
(45) Date of publication and mention of the grant of the patent: **01.04.2015**
(21) Application number: 10719223.9
(22) Date of filing: 14.05.2010
(51) Int. Cl.: G01N 33/50

(54) **PLATFORM TECHNOLOGIES FOR SPONTANEOUSLY OCCURRING DISEASES**
PLATTFORMTECHNOLOGIEN FÜR SPONTAN AUFTRETENDE KRANKHEITEN
TECHNOLOGIES DE PLATEFORME POUR DES MALADIES SE PRODUISANT SPONTANÉMENT

(30) Priority: 11.06.2009 US 186342 P; 14.05.2009 US 178391 P
(43) Date of publication of application: 21.03.2012
(73) Proprietor: Oatmeal Biotechnologies Group, L.L.C., Woodside, CA 94062 (US)
(72) Inventor: FRANK, Matthew, Woodside CA 94062 (US)
(74) Representative: Roques, Sarah Elizabeth
(86) International application number: PCT/US2010/035019
(87) International publication number: WO 2010/132847

(56) References cited:
- EP-A2- 0 091 784
- KENNETH M RASSNICK ET AL: "In vitro and in vivo evaluation of combined calcitriol and cisplatin in dogs with spontaneously occurring tumors" CANCER CHEMOTHERAPY AND PHARMACOLOGY, SPRINGER, BERLIN, DE, vol. 62, no. 5, 2 February 2008 (2008-02-02), pages 881-891, XP019625569 ISSN: 1432-0843
- THAMM DOUGLAS H ET AL: "Intralesional lipid-complexed cytokine/superantigen immunogene therapy for spontaneous canine tumors." CANCER IMMUNOLOGY IMMUNOTHERAPY, vol. 52, no. 8, August 2003 (2003-08), pages 473-480, XP002587654 ISSN: 0340-7004
- SPUGNINI ENRICO P ET AL: "Patterns of tumor response in canine and feline cancer patients treated with electrochemotherapy: preclinical data for the standardization of this treatment in pets and humans" JOURNAL OF TRANSLATIONAL MEDICINE, BIOMED CENTRAL, LONDON, GB, vol. 5, no. 1, 2 October 2007 (2007-10-02) , page 48, XP021037631 ISSN: 1479-5876
- PAOLONI MELISSA C ET AL: "Launching a novel preclinical infrastructure: comparative oncology trials consortium directed therapeutic targeting of TNFalpha to cancer vasculature." PLOS ONE 2009 LNKD- PUBMED:19330034, vol. 4, no. 3, March 2009 (2009-03), page E4972, XP002587656 ISSN: 1932-6203
- HANSEN K ET AL: "Spontaneous and genetically engineered animal models - use in preclinical cancer drug development" EUROPEAN JOURNAL OF CANCER, PERGAMON PRESS, OXFORD, GB LNKD- DOI:10.1016/J.EJCA.2003.11.031, vol. 40, no. 6, 1 April 2004 (2004-04-01), pages 858-880, XP004500013 ISSN: 0959-8049
- PAOLONI MELISSA ET AL: "Science and society - Translation of new cancer treatments from pet dogs to humans" NATURE REVIEWS CANCER, vol. 8, no. 2, February 2008 (2008-02), pages 147-156, XP002599784 ISSN: 1474-175X cited in the application
- MACK GEORGE S: "Clinical trials going to the dogs: Canine program to study tumor treatment, biology" JOURNAL OF THE NATIONAL CANCER INSTITUTE (CARY), vol. 98, no. 3, February 2006 (2006-02), pages 161-162, XP002587655 ISSN: 0027-8874
- SCOTT HAFEMAN ET AL: "Evaluation of liposomal clodronate for treatment of malignant histiocytosis in dogs" CANCER IMMUNOLOGY, IMMUNOTHERAPY, SPRINGER, BERLIN, DE, vol. 59, no. 3, 16 September 2009 (2009-09-16), pages 441-452, XP019778759 ISSN: 1432-0851
- RUSK A ET AL: "Preclinical evaluation of antiangiogenic thrombospondin-1 peptide mimetics, ABT-526 and ABT-510, in companion dogs with naturally occurring cancers" CLINICAL CANCER RESEARCH, THE AMERICAN ASSOCIATION FOR CANCER RESEARCH, US LNKD- DOI:10.1158/1078-0432.CCR-06-0109, vol. 12, no. 24, 15 December 2006 (2006-12-15), pages 7444-7455, XP002460385 ISSN: 1078-0432 cited in the application

## Description

### CROSS-REFERENCE TO RELATED APPLICATIONS

This application claims priority to U.S. provisional patent applications 61/178,391, filed on May 14, 2009, and 61/186,342, filed on June 11, 2009.

### BACKGROUND OF THE INVENTION

The endeavor to improve human lives includes the discovery of new biological pathways and mechanisms of action as well as new treatment and diagnostic modalities. The discovery of new drugs, compounds, methods, or the combinations of any of the foregoing, for combating various diseases, such as cancer, is difficult due to regulatory mandates as well as time and cost considerations. A comprehensive study of multiple treatments is very hard to achieve in human clinical trials for the same reasons. These reasons act as real life barriers that impede the efforts of companies, non-profit organizations, and individuals to save human lives and/or improve living conditions of humans who are afflicted with various diseases. What is needed is an improved system for studying various diseases such that a combination of factors can be investigated to determine the most optimal biological and/or physiological response and outcome. Such system can be utilized to translate the information to generate new or improved drugs, compounds and treatment protocols to provide the maximally efficient use of medical and scientific efforts to help individuals with various diseases, such as spontaneously occurring diseases that involve host-induced responses (e.g., diabetes, cancer, autoimmune, neurological, allergic diseases).

Spontaneously occurring diseases, such as diabetes, have been observed in companion animals, such as dogs and cats (Hoenig M, Mol. Cell. Endocrinol. 197: 221-229 (2002)). For example, Davison et al. describes studies performed on autoantibodies to GAD65 and IA-3 in spontaneously occurring diabetes mellitus (Davison LJ et al., Veterinary Immunology and Immunopathology, 126: 83-90 (2008)). Hoenig et al. described a qualitative assay for beta cell antibodies in dogs with diabetes in Veterinary Immunology and Immunopathology, 32: 195-203 (1992)). Other naturally occurring diseases in dogs have been described in various references, e.g., Tsai et al., Mamm. Genome, 18:444-451 (2007).

In addition to diabetes, other spontaneously occurring diseases have been observed, such as cancer and autoimmune disease. Paoloni et al. describe the integration of the study of dogs with naturally occurring cancer with the study of human cancer biology to identify cancer-associated genes, study environmental risk factors, understand tumor biology and progression and evaluate and develop novel cancer therapeutics. (Nature, 8: 147-156 (2008)). The Canine Comparative Oncology and Genomics Consortium (CCOGC) is the result of many collaborative efforts to use the dog as a model of naturally occurring cancer for investigating cancer research in efforts to better both humans and dogs. Nature Biotechnology 24(9): 1065-1066 (2006). Examples of cancers that dogs naturally develop include: non-Hodgkin lymphoma, osteosarcoma, melanoma, prostate carcinoma, lung carcinoma, head and neck carcinoma, mammary carcinoma, and soft-tissue carcinoma. *Ibid.* Trials in pet dogs have been reported to help better define the safety and activity of new anticancer agents, assist in the identification of relevant biomarkers associated with the response or exposure to these anticancer drugs, and may allow rational development of combination strategies to improve the success of these new drugs in human clinical trials. *Ibid.* Candolfi et al describe the use of adenoviral-mediated gene transfer into dogs that spontaneously develop glioblastoma mutliforme (GBM) (Candolfi M et al, Neurosurgery 60: 167-178 (2007)). Paolini et al. reported that the Comparative Oncology Trials Consortium (COTC) evaluated a targeted AAV-phage vector delivering tumor necrosis factor (RGD-A-TNF) to αV integrins on tumor endothelium. PLoS ONE 4(3): e4972 (2009).

EP 0091784 discloses the analysis of various chemotherapeutic agents on dogs with various spontaneous tumors. Rassnick K et al, Cancer Chemother. Pharmacol. 62:881-891 (2008) discloses *in vitro* and *in vivo* evaluation of combined calcitrol and cisplatin in dogs with spontaneously occurring tumors. Thamm D et al, Cancer Immunol. Immunother. 52:473-480 (2003) discloses intralesional lipid-complexed cytokine/superantigen immunogene therapy for spontaneous canine tumors. Spugnini E *et al,* J. Trans. Med. 5:48 (2007) discloses patterns of tumor response in canine and feline cancer patients treated with electrochemotherapy for standardisation of this treatment in pets and humans.

The invention described herein provides platform technologies for studying spontaneously occurring cancer that can be translated into therapeutic treatments and diagnostic methods.

### BRIEF SUMMARY OF THE INVENTION

The invention provides for platform technologies for investigating biological pathways, the effects (e.g., synergistic effects) of various combination of agents that affect biological and/or physiological pathways, underlying mechanisms of action, biological participants in complex physiological conditions and other parameters that can be useful for development of agents for treatment, diagnosis, or prophylaxis of cancer.

Accordingly, in one aspect, the invention provides for methods for identifying a combination of anti-cancer agents with synergistic effects comprising: (1) administering two or more anti-cancer agents to a companion animal with a spontaneously occurring cancer; (2) monitoring the companion animal for a biological and/or physiological effect; and (3), identifying a combination of anti-cancer agents with synergistic effects when the biological and/or physiological effects are synergistic, wherein the anti-cancer agents comprise bisphosphonates and cationic CpG. In one embodiment, the agents are clodronate and cationic CpG.

In any of the aspects or embodiments of this invention, the companion animal is a dog. The dog can be a purebred dog or a mongrel dog. The dog can have a homogeneous genetic background or a heterogeneous genetic background.

In any of the aspects or embodiments of this invention, the companion animal is a cat. The cat can be a purebred or a mongrel. The cat can have a homogeneous genetic background or a heterogeneous genetic background.

We describe a companion animal model system for identifying a treatment modality for treatment in humans comprising a combination of compositions that have a higher probability of success for identifying the treatment modality than a standard model.

In another aspect, the invention provides for methods for identifying an anti-cancer agent comprising: (a) procuring companion animal model system for testing the agent wherein the companion animal model system has a spontaneously occurring cancer; (b) administering the agent to the companion animal model system; (c) monitoring more than one cancer antigen in the companion animal model system for biological and/or physiological effects; and (d) identifying the agent as anti-cancer based on the biological and physiological effects. In one embodiment, the companion animal is a dog or a cat. In another embodiment, the cancer antigen is not a glioblastoma multiforme antigen.

We describe methods of targeting multiple antigens associated with or suspected of being associated with cancer in a human comprising: (a) procuring companion animal model system for testing the agent wherein the companion animal model system has a spontaneously occurring cancer; (b) administering to the companion animal model system one or more agents that is suspected of having anti-cancer effects; (c) monitoring the effects of the agent on the companion animal model system; and (d) administering the same agent to the human if the agent has an anti-cancer effect in the companion animal model system.

We describe methods of improving timing and/or cost for obtaining regulatory approval on an agent for a disease comprising: (a) identifying companion animal model system for the disease wherein the companion animal model system has a spontaneously occurring version of the disease; (b) administering the agent to the companion animal model system; (c) monitoring the animal for biological and physiological effects; (d) determining the effects of the agent on the disease and (e) documenting the effects of the agents on media that is suitable for submission to a regulatory agency.

### BRIEF DESCRIPTION OF THE FIGURES

Figure 1 depicts results which show that once weekly i.v. administration of 200 ul LC to C57B1/6 mice with established s.c. MCA-205 (sarcoma) tumors produced significant inhibition of tumor growth.
Figure 2 depicts results which shows a dog with STS treated with a series of treatments with LC alone experienced significant spontaneous tumor regression beginning after the third LC administration.
Figure 3 depicts results which show that twenty-four hours after i.v. administration of LC in tumor-bearing mice, CD11b⁺/Gr-1⁺ MSC were enumerated in spleen, blood, and tumor tissues and that significant MSC depletion occurred in blood.
Figure 4 depicts results which show that the antitumor activity of LC was almost completely eliminated in CD8^{-/-} mice, whereas the activity of LC was only partially inhibited in CD4^{-/-} mice. Controls also included mice treated with PBS containing liposomes (lip control).
Figure 5 depicts results from experiments which tested whether MSC depletion using LC could enhance vaccine responses, using humoral immune responses as the readout.

### DETAILED DESCRIPTION OF THE INVENTION

The invention provides platform technologies for studying various aspects of biological pathways, physiological conditions and/or responses, and underlying mechanisms of action for cancer, such as spontaneously occurring cancer. Such knowledge can be further used for translational medicine for various purposes, including but not limited to developing treatments, diagnostic methods or kits; identifying new pathways, identifying compounds or agents (and combinations thereof) for therapeutic or prophylactic purposes, and/or identifying new disease targets.

Generally, companion animals with spontaneously occurring diseases are useful for gathering data on various treatment modalities and combinations. Since companion animals are not kept under laboratory conditions (i.e., with limited exposure to every day environmental factors, and exposed to a controlled set of conditions), the use of such animals is one distinguishing factor from the other studies (e.g., beagle studies) using canines kept under laboratory conditions. Furthermore, the diseases are not being induced by reagents under laboratory conditions, i.e., the diseases develop spontaneously. Thus, the benefit of this platform is that it is more reflective of what happens to humans than laboratory animals which have been induced to develop a particular disease or condition.

Non-human companion animals, such as dogs, spontaneously develop diseases that mirror human diseases. As such, the use of companion animals that develop spontaneously occurring diseases can provide additional benefits by decreasing the time needed to gather scientific data for regulatory approval, decrease the cost associated with such data gathering and increase the amount of scientific data that can be obtained. The use of animal models with spontaneously occurring diseases permits testing of one or more parameters (such as type of antigen(s), combination of antigens, combination of agents, location of delivery, etc.) that would otherwise not be permitted under FDA regulations. Furthermore, companion animals are also helped by potential discoveries that could cure or treat their spontaneously occurring diseases.

We describe a companion animal model system as a platform technology for identifying a treatment modality for treatment in humans comprising a combination of compositions that have a higher probability of success for identifying the treatment modality than a standard model. The companion animal can be any animal that are companions to humans, preferably exposed to the same environmental factors (e.g., air, water) as their humans. In one aspect, the companion animal is an animal whose genome is has been determined either partially or fully. Use of genomic information (e.g., at the nucleic acid level, protein or metabolic level) is useful in these platform technologies. Non-limiting examples of companion animals who share similar environmental factors to their humans and have their genome partially or fully sequences include dogs and cats.

The use of the platform technologies described herein can provide 20-50 fold reduction in the time and/or cost for translational medicine by exploiting the synergies between multiple platforms as well as between multiple antigens and any combination thereof. As described in greater detail herein, the platform technologies can be applied to different subject matter that traditionally have faced difficulties in human trials due to costs, regulatory constraints, timing, size of trials and other road blocks for advancement of science. This subject matter includes, but is not limited to, vaccines (e.g., tolerizing vaccines), cationic lipid CpG, quorum sensing and autoinducer in infectious diseases, multiplexing pathology from biological samples (e.g., urine, saliva, blood or plasma), diagnostic techniques for rapid diagnosis and marker multiplexing technology.

### General Techniques

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed., 1984); Animal Cell Culture (R.I. Freshney), ed., 1987); Methods in Enzymology (Academic Press, Inc.); Handbook of Experimental Immunology (D.M. Weir & C.C. Blackwell, eds.); Gene Transfer Vectors for Mammalian Cells (J.M. Miller & M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991) and Short Protocols in Molecular Biology (Wiley and Sons, 1999). Other useful references include Harrison's Principles of Internal Medicine (McGraw Hill; J. Isseleacher et al., eds.), Dubois' Lupus Erythematosus (5th ed.; D.J. Wallace and B.H. Hahn, eds.; Williams & Wilkins, 1997), Textbook of Veterinary Internal Medicine: Diseases of the Dog and Cat (Stephen Ettinger, ed., W.B. Saunders Company; 5th edition (January 15, 2000)); and Kirk's Current Veterinary Therapy XIV (Bonagura et al, Saunders; 14 edition (July 10, 2008)).

### Definitions

As used herein, the singular form "a", "an", and "the" includes plural references unless indicated otherwise. For example, "an" antigen includes one or more antigens.

An "individual" is a vertebrate, preferably a mammal, more preferably a human. Mammals include, but are not limited to, farm animals, sport animals, pets, companion animals, primates, mice and rats. In one embodiment, an individual is a human.

A "companion animal" is a non-human animal that resides in the same household as their human owners for companionship. Companion animals generally are exposed to the same environmental factors as humans (e.g., water, air, carcinogens, allergens, etc.). Non-limiting examples of a companion animal include dogs and cats. In one aspect, a companion animal is not subjected to laboratory conditions (e.g., with limited exposure to every day environmental factors and exposed to a controlled set of conditions).

As used herein, "spontaneous occurring" or "spontaneously occurring" (or naturally occurring) diseases are diseases which involve host-induced disease states. Host-induced disease states refer to the host mounting some type of biological or physiological response in certain circumstances. In one embodiment, host-induced disease states do not include virally-induced states wherein the virus is the causative agent for the transformation. In another embodiment, "spontaneously occurring" includes biological and/or physiological conditions or responses brought on by viruses. For example, a mouse or rat can be induced to have cancer by injecting the mouse or rat with certain chemicals. The cancer-ridden mouse or rat would not be considered to have "spontaneous occurring cancer."

"Synergy" as used to describe biological and/or physiological effects of a combination of agents or treatment modalities refers to one or more effects that are greater than additive of each agent or treatment modality by itself. For example, if administration of one agent results in a 10% antibody increase and administration of another agent results in a 15% antibody increase, then a synergistic effect would be greater than 25% antibody response. In some embodiments, a synergistic effect is 1%, 2%, 3%, 4%, 5%, 6%, 7%, 8%, 9%, or 10% more than additive effect. In other embodiments, a synergistic effect is 15%, 20%, 25%, 30%, 35%, 40%, 45%, 50%, 55%, 60%, 65%, 70%, 75%, 80%, 85%, 90%, 95%, or 100% more than additive effect. In other embodiments, a synergistic effect is or 125%, 150%, 200%, 300%, 400%, or 500% more than additive effect. In other embodiments, a synergistic effect can be a 2-fold, 3-fold, 4-fold, 5-fold, 6-fold, 7-fold, 8-fold, 9-fold, or 10-fold increase over additive effect.

"Synergy" can also be used to describe a decrease in biological and/or physiological effects (e.g., autoimmune response) in addition to an increase in biological and/or physiological effects (e.g., antibody production). For example, if administration of one agent results in a 10% decrease in autoimmune response (e.g., antinuclear antibodies for systemic lupus erythematosus) and administration of another agent results in a 15% decrease, then a synergistic effect would be a decrease of more than 25% autoimmune response. In some embodiments, a synergistic effect is 1%, 2%, 3%, 4%, 5%, 6%, 7%, 8%, 9%, or 10% less than additive effect. In other embodiments, a synergistic effect is 15%, 20%, 25%, 30%, 35%, 40%, 45%, 50%, 55%, 60%, 65%, 70%, 75%, 80%, 85%, 90%, 95%, or 100% less than additive effect. In other embodiments, a synergistic effect is or 125%, 150%, 200%, 300%, 400%, or 500% less than additive effect. In other embodiments, a synergistic effect can be a 2-fold, 3-fold, 4-fold, 5-fold, 6-fold, 7-fold, 8-fold, 9-fold, or 10-fold decrease over additive effect.

"Agent" can refer any composition of matter, whether it is naturally occurring or synthetic. Non-limiting examples of an agent include: small molecules, antibodies, naturally occurring protein and fragments thereof (e.g., soluble receptors like Axl, EGF, or VEGF or other involved with the growth factors), recombinant proteins and fragments thereof, fusion molecules (e.g., fusion proteins), synthetic molecules, lipids, nucleic acids, and carbohydrates.

"Biological and/or physiological effect" refers to the effect of an agent on an individual's biological parameters or physiological parameters. Non-limiting examples of biological parameters include: cytokine profile and/or production, immune response, immune parameters such as antibody response, Th1 or Th2 or Th17 responses, genomic profile and its changes, antigen profile and its changes, lipid profiles, fatty acid and cholesterol profiles and toxicity profiles. Non-limiting examples of physiological parameters include parameters associated with a system, e.g., cardiovascular system. Such cardiovascular parameters can include, but are not limited to, cardiac health, pulmonary artery occlusion, coronary perfusion pressures; cardiac output, pulmonary, systemic vascular resistances. In other embodiments, the physiologic parameters can include, but are not limited to, blood gas and saturation measurements, oxygen delivery, oxygen utilization, renal capacity, and processing and functional capability of organs (e.g., liver for toxins, pancreas for insulin production, etc.).

"Disease" refers to an abnormal condition of an individual that can impair bodily functions, and is commonly associated with specific symptoms. It may be caused by external factors, such as invading pathogens, or it may be caused by internal dysfunctions, such as autoimmune diseases. "Disease" also encompasses various states and degrees of each disease. For example, the development of a malignant growth is a disease state of cancer. Metastasis is another disease state of cancer. All the symptoms and/or signs reported to be associated with the development of the disease does not necessarily need to be present in an individual for any given disease.

"Antigen," as used herein, refers broadly to any substance that can be recognized by an organism's immune system. In one aspect, antigens can induce the production of antibodies. Antigens are typically proteins or polysaccharides. Antigens include, but are not limited to, parts (coats, capsules, cell walls, flagella, fimbrae, and toxins) of bacteria, viruses, and other microorganisms. Antigens do not necessarily have to elicit an immune response by themselves alone. Antigens encompass immunogens, which do elicit an immune response (e.g., antibody response). Types of antigens include, but are not limited to, exogenous antigens (antigens that have entered the body from the outside, for example by inhalation, ingestion, or injection), endogenous antigens (antigens that have been generated within the cell, for example, as a result of normal cell metabolism, or because of viral or intracellular bacterial infection), autoantigens, tumor antigens and allergic antigens.

An "autoantigen" is usually a normal protein or complex of proteins (and sometimes DNA or RNA) that is recognized by the immune system of patients suffering from a specific autoimmune disease. These antigens should, under normal conditions, not be the target of the immune system, but, due to mainly genetic and environmental factors, the normal immunological tolerance for such an antigen has been lost in these patients.

As used herein, "treatment" is an approach for obtaining beneficial or desired results, preferably including clinical results. For example, in the context of this invention, one desired results would be the halt of the growth of cancer cells. Treatment does not necessarily require that the disease be eradicated or that the individual with the disease be cured.

"Receiving treatment" includes initial treatment and/or continuing treatment.

"Therapy" includes both prophylactic therapy (i.e., before disease occurrence) and therapeutic treatment (i.e., after disease occurrence).

"Beneficial effect" refers to a biological or physiological effect on the individual (e.g., human or companion animal) that improves the well-being of the individual. Non-limiting examples of a beneficial effect include: reduction of cancerous tumors or nodules, reduction in the number of malignant cells, increased antibody production against cancer or pathogens, secretion of cytokines that assist in eliminating cancer cells and/or pathogens, decrease in the amount of immune reaction against self-molecules, reduction in the autoimmune response, palliating symptoms of a disease, palliating undesired pain in an individual, increasing the comfort level of an individual, increasing the robustness of the individual's immune system, and reconstituting an individual's immune system.

As used herein, "combination" refers to all the possible variations for the combination of any agent, antigen, composition, compound, adjuvant, etc. with each other. This includes the use of more than one of any one agent, antigen, composition, adjuvant and the like within its own group (e.g., multiple agents) or with other groups. For example, "combination" contemplates the use of one agent with one adjuvant or two compositions with several adjuvants.

### Compositions of Treatment Modalities

The invention provides platform technologies that utilize a companion animal model system of spontaneously occurring cancer to investigate aspects of human cancer. Companion animal models that may be used include any animal who resides with humans. In this manner, the companion animal is exposed to similar environmental factors as their human co-inhabitants. Such environmental factors include, but are not limited to, breathing the same air, drinking the same water, exposure to the same household contents (e.g., carpets, cleaners, etc.) Unlike laboratory animals that are typically used for experiments (e.g., mice and rats), companion animals are exposed to the factors that a human is and, as such, provide a more accurate background for correlation for human diseases and/or physiological conditions. Any treatments that are beneficial for humans can be used to help the companion animal as well, which includes not only treating the disease and/or physiological condition, but also to improve their quality of life.

We describe that the examination of multiple modalities is conducted using a canine model system. In one embodiment, multiple modalities can refer to the use of multiple antigens in the system. The study of a single antigen may not provide sufficient insight into a biological system for generating an efficient immune response. For example, the identification of a single antigen associated with prostate cancer, e.g., prostatic acid phosphatase, may mount an immune response but the identification of other antigens would provide additional, even synergistic, immune response to combat prostate cancer. The study of multiple antigens in human clinical trials is not feasible due to regulatory constraints (e.g., FDA approval), cost, time, and/or other biological barriers. In this regard, the use of a canine model system is useful for examination of multiple antigens since dogs spontaneously develop prostate cancer. One of skill in the art can use the canine model system to examine multiple antigens, for example, cancer antigens, to identify novel antigens that can be used for targets (e.g., antibodies against the antigen, small molecules, etc.). In addition, the use of canine model system can assist to identify new pathways and/or biological niches that the antigen is associated and be utilized as a basis for additional therapies.

We describe that multiple modalities can refer to the use of one or more antigens plus one or more adjuvants. The term "adjuvant" is well-known in the art. It commonly refers to a pharmacological or immunological agents that can modify the effect of other agents (e.g., drugs or vaccines) while having few if any direct effects when given by themselves. An adjuvant can be an immunological adjuvant, which can modify or augment the effects of a vaccine by stimulating the immune system to respond to the vaccine more vigorously, and thus providing increased immunity to a particular disease. Non-limiting examples of immunological adjuvants include: alum, Freud's complete adjuvant, Freud's incomplete adjuvant, Ribi adjuvant, aluminum salts, and immunomodulatory polynucleotides (e.g., CpG-containing polynucleotides). An adjuvant can also be a pharmaceutical adjuvant which have few or no pharmacological effects by themselves, but may increase the efficacy or potency of other drugs when given at the same time. A non-limiting example of this is caffeine, which has minimal analgesic effect on its own, but may have an adjuvant effect when given with paracetamol (acetaminophen). Adjuvant can also refer to additional therapy in the cancer therapy context, for example, in chemotherapy. In this context, adjuvant therapy refers to additional treatment, usually given after surgery where all detectable disease has been removed, but where there remains a statistical risk of relapse due to occult disease. In a non-limiting example, radiotherapy or chemotherapy is can be given as adjuvant treatment after surgery for a breast cancer.

The use of "reverse adjuvants" is encompassed by the term "adjuvant." Reverse adjuvants can have tolerizing effects when used with a tolerizing vaccine (*see, e.g.,* Ho et al, J. Immunology 175: 6226-6234, 2005). One example of a reverse adjuvant is GpG oligonucleotide which has suppressive effects in contrast with CpG oligonucleotides, which tend to have immunostimulatory properties (*see, e.g.,* Ho et al, J. Immunology 171: 4920-4926, 2003).

The combination of various antigens and adjuvants and their effect on various spontaneously occurring diseases, has been difficult to study in human trials for reasons discussed above. Using mice or rats as animal models does not provide as accurate of information as using canine model system of spontaneously occurring diseases since the genetic translation to humans and disease progression does not parallel as closely as dogs to humans. (Tsai et al., Mamm. Genome, 18:444-451 (2007). As such, the use of canine model system as a platform technology for examining spontaneously occurring diseases allows for one of skill in the art to identify a treatment modality with a greater probability of success than using a standard mouse model where the diseases are induced.

Various types of adjuvants can be studied using the canine model system disclosed herein. In one embodiment, adjuvants that act through the toll-like receptor (TLR) agonists can be studied using the platform technology of canine model system of spontaneously occurring diseases. Various TLR include, but are not limited to, TLR 1, 2, 3, 4, 5, 6, 7, 8, and 9. One example is CpG-containing compounds that act through the TLRs. Such adjuvants have been tested in the context of hepatitis B. Other non-limiting examples of adjuvants that can be studied include keyhole limpet hemocyanin (KLH) and MF59.

In another aspect, the platform technologies described herein allows one of skill in the art to explore the use of multiple modalities against a heterogeneous genetic background. One of skill in the art will appreciate that there are various degrees of heterogeneity and homogeneity in genetic backgrounds. On one end of spectrum, homogeneous genetic backgrounds are commonly seen in cloned animals or animals such as mice that have been inbred for many generations such that their genetic background is the same as the next mouse.

Further down the spectrum are heterogeneous animals (e.g., with less degree of homogeneity than cloned animals), such as purebred dogs. Although they are purebred, the dogs have slightly different genetic code from each other but yet they retain the same morphological traits that characterize them as being that particular purebred. Dogs are unique among mammalian species in that they can show differences in morphological traits (such as height, weight, shape) and yet within breed, exhibit traits that are inherited within a narrow range. For example, purebred chihuahua dogs are generally +/- 6 inches of each other at the shoulder. Ostrander et al., Am J Hum Genet 61:475-480 (1997). Even further down the spectrum are even more heterogeneous dogs which are not purebred but instead are mongrels. In one embodiment, heterogeneous animals do not include non-obese diabetic (NOD) mouse. It is against this backdrop of heterogeneous genetic background that different treatment modalities are explored. The heterogeneous nature of the dogs does not necessarily allow one of skill in the art to predict *a priori* what the biological response will be, and even more so in the case where multiple treatment modalities (e.g., multiple antigens) are utilized. The foregoing is equally applicable with other companion animals, such as cats.

### Advantages of Using a Spontaneously Occurring Disease Model

The use of spontaneously occurring disease model is beneficial in various aspects. In one aspect, the immune system of the disease model is kept relatively intact as compared to animal model of disease where the animal has been induced to have the disease. In the latter case, artificial induction of diseases throws off the balance of the immune system, causes the immune system (including various immune cells such as T cells, B cells, neutrophils, macrophages, regulatory Tcells, NK cells, NKT cells) and the interactions between immune cells and various branches of the immune system to be perturbed by the artificial induction of the diseases. Accordingly, the invention provides a platform technology that allows for the study of cancer without the immune dysregulation associated with the artificial induction of the disease. This provides more meaningful findings which facilitates the discovery and/or identification of new pathways, mechanisms of action, biological participants (e.g., cellular receptors or cell types) in these pathways or mechanisms and further understanding to the underpinnings of cancer.

We describe the use of the platform technology for the identification of one or more biomarkers associated with various physiological states and diseases. In some instances, biomarker can refer to the presence or absence of one or more genes or proteins, various isoforms of genes or gene splicing and their product(s), single nucleotide polymorphisms, gene expression profiles, proteomic profiles or metabolomic profiles. In some non-limiting examples, multiplexing biomarkers are used for screening, staging, imaging, diagnosing and/or monitoring the response to various therapies. For example, changes to expression of one or more genes, metabolome and epigenetic changes are contemplated. In one non-limiting example, methylation patterns on gene chips can be used to study normal vs. abnormal methylation patterns for various diseases/disease states. Another non-limiting example is the use of magnetic arrays that can house multiple biomarkers (e.g., 15-18 biomarkers) and are detectable at low amounts (e.g., 1 pg/ml). Another non-limiting example is the use of aptamers where hundreds of biomarkers can be assessed simultaneously or nearly simultaneously. One of skill in the art can utilize the screening, staging, imaging, diagnosing and/or monitoring in combination with treatment protocols and the refinement of any therapies that are being contemplated. One of skill in the art, e.g., a physician, can modify the therapy as to most effectively prevent, delay the development of, ameliorate the symptoms of, or treat the disease or physiological condition.

### Cancer

The use of companion animals with spontaneously occurring cancer allows for one of skill in the art to not only seek durable cures for companion animals but also to use the companion animal as a model for studying scientific aspects of cancer (including spontaneously occurring cancers) in humans, which may lead to discoveries for treatments and therapies for various types of cancers for mankind.

Incidence rates of human and companion animal cancers vary considerably. In some cases, human cancers are not commonly found in pets, and comparative oncology in not practical. In other cases, tumors in companion animals closely resemble their human counterparts and in some cases may occur more frequently, affording the opportunity to study diseases that are rare in human cancer patients.

Companion animals such as dogs develop various types of spontaneously occurring cancers. Common cancers include, but are not limited to, bone cancer (e.g., osteosarcoma), lymphoma (e.g., non-Hodgkin lymphoma), hemangiosarcoma, other sarcomas, mammary cancer, testicular cancer, mast cell cancer, nasosinal cancer, bladder cancer, head and neck cancer, prostate cancer, melanoma, leukemia, brain cancer, lung carcinoma, and soft-tissue carcinoma. Some breeds develop certain cancers more often than other breeds. For example, hemangiosarcomas, an aggressive cancer that arises from the blood vessels, are seen more in German Shepherds, Golden Retrievers, Boxers, and English Setters than other breeds. In one aspect, one of skill in the art can observe the differences in the cancer progression when different biological procedures that normally would be applied to the companion animal are done. For example, prostate cancer progression can be observed in dogs who have been neutered and compared to prostate cancer progression to dogs whose owners have chosen to not have them neutered.

In one aspect, the use of purebred dogs allows for the study of a more homogeneous genetic background and comparison with mongrel dogs with heterogeneous genetic background. The use of various genetic backgrounds of companion animals, such as dogs, permits the identification of various antigens or biomarkers that are associated with cancer. The resulting information gleaned from such studies can be translated into diagnostics or therapies for humans by using antigens as targets for drug discovery or immunotherapy and/or by using biomarkers in imaging techniques.

Companion animals with spontaneously occurring cancer can be used to examine the effects of a combination of anti-cancer agents to identify a combination that produces synergistic effects. The combination of agents can be two or more agents, for example, 3, 4, 5, 6, 7, 8, 9, or 10 agents. The agents can be given at the same time or in two or more administrations. The dosage of each agent can be same or varied, especially when using a group of companion animals with spontaneously occurring cancers where a range of dosage of agents tested can indicate which combination results in most efficacious biological response.

The anti-cancer agents of the invention comprise bisphosphonates and cationic CpG. Various classes of anti-cancer agents can be used. Non-limiting examples include: alkylating agents, antimetabolites, anthracyclines, plant alkaloids, topoisomerase inhibitors, podophyllotoxin, antibodies (e.g., monoclonal or polyclonal), tyrosine kinase inhibitors (e.g., imatinib mesylate (Gleevec® or Glivec®), hormone treatments, soluble receptors and other antineoplastics.

Alkylating agents can alkylate many nucleophilic functional groups under conditions present in cells. Cisplatin and carboplatin, and oxaliplatin are alkylating agents. They impair cell function by forming covalent bonds with the amino, carboxyl, sulfhydryl, and phosphate groups in biologically important molecules.

Anti-metabolites resemble purine ((azathioprine, mercaptopurine)) or pyrimidine and prevent these substances from becoming incorporated in to DNA during the "S" phase of the cell cycle, stopping normal development and division. They also affect RNA synthesis.

Plant alkaloids and terpenoids are derived from plants and block cell division by preventing microtubule function. Since microtubules are vital for cell division, without them, cell division cannot occur. Some non-limiting examples are vinca alkaloids and taxanes.

Vinca alkaloids bind to specific sites on tubulin, inhibiting the assembly of tubulin into microtubules (M phase of the cell cycle). The vinca alkaloids include: vincristine, vinblastine, vinorelbine, and vindesine.

Podophyllotoxin is a plant-derived compound which has been reported to help with digestion as well as used to produce two other cytostatic drugs, etoposide and teniposide. They prevent the cell from entering the G1 phase (the start of DNA replication) and the replication of DNA (the S phase).

Taxanes as a group includes paclitaxel and docetaxel. Paclitaxel is a natural product, originally known as Taxol and first derived from the bark of the Pacific Yew tree. Docetaxel is a semi-synthetic analogue of paclitaxel. Taxanes enhance stability of microtubules, preventing the separation of chromosomes during anaphase.

Topoisomerase inhibitors are also another class of anti-cancer agents that can be used. Topoisomerases are essential enzymes that maintain the topology of DNA. Inhibition of type I or type II topoisomerases interferes with both transcription and replication of DNA by upsetting proper DNA supercoiling. Some type I topoisomerase inhibitors include camptothecins: irinotecan and topotecan. Examples of type II inhibitors include amsacrine, etoposide, etoposide phosphate, and teniposide. These are semisynthetic derivatives of epipodophyllotoxins, alkaloids naturally occurring in the root of American Mayapple (Podophyllum peltatum).

Antineoplastics include the immunosuppressant dactinomycin, doxorubicin, epirubicin, bleomycin, mechlorethamine, cyclophosphamide, chlorambucil, ifosfamide. The antineoplastic compounds generally work by chemically modifying a cell's DNA.

Soluble receptors can include the extracellular portion of receptors known to bind to growth factors and especially growth factors that are associated with cancer. Non-limiting examples are: Axl, VEGF, and EGF. The soluble receptors can be recombinant/synthetic or naturally occurring receptors (e.g., purified or concentrated preparation). The extracellular portion of receptors can also be fused to portions to promote half-life and other desirable pharmacokinetics to create fusion proteins.

### Osteosarcoma

Osteosarcoma is a relatively rare form of cancer afflicting a disproportionate percentage of children, with an annual incidence of 900 new patients per year including 400 < 20 years old. Though rare, it is the 6^{th} leading form of cancer in children under the age of 15, about 3% of all childhood cancers. The current standard of care is amputation or limb-salvage orthopedic surgery combined with chemotherapy (high dose methotrexate with leucovorin rescue, intra-arterial cisplatin, adriamycin, ifosfamide, etoposide, and muramyl tri-peptide). Survival rates have improved since the 1960s when the only treatment option was amputation and only 5-20% of diagnosed patients survived more than 2 years, but despite improvements through chemotherapy the survival rate for osteosarcoma remains among the lowest among pediatric cancers. The current 5-year survival rate for non-metastatic osteosarcoma patients is > 70% while for patients with metastases the rate is approximately 30%. Progress toward improved treatment options for this young population is slowed by its rare incidence and the resultant challenges in patient accrual for clinical studies.

In contrast to human incidence, osteosarcoma is a relatively common cancer in larger breeds of dogs (>60 pounds), particularly in Great Dane, Wolfhound, and Rottweiler. The incidence of osteosarcoma is 3-4% of all canine cancers, afflicting up to 10,000 dogs per year in North America. Human and canine osteosarcoma share common features of anatomical distribution and metastasis. In both species, >75% of cases occur in long bones (distal radius > proximal humerus; distal femur > tibia), predominantly in males (2:1). The high metastatic rate in dogs (90%) is comparable to that in humans (80%), and sites of metastasis have a similar hierarchy of lung>bone>soft tissue. Furthermore, primary osteosarcoma and metastases are histologically indistinguishable between human and canine patients. Like humans, dogs also respond to chemotherapy- treatment with cisplatin, doxorubicin, or carboplatin following amputation produces a mean survival time of 9-11 months, a significant improvement over the median survival of 3-4 months following amputation alone. Given the shared histology, metastatic pattern, and chemotherapy responsiveness, canine osteosarcoma offers an excellent model for testing alternative therapies. With a higher incidence and more rapid progression, clinical trials can be recruited and completed more rapidly in dogs, informing new therapeutic strategies for both human and canine patients.

### Soft Tissue Sarcomas

Soft tissue sarcomas are a diverse group of tumors derived from mesenchymal tissues (e.g. connective tissue, fibrous tissue, muscle). They account for less than 1% of all new cancer cases per year; in 2006 there were approximately 9,500 new cases diagnosed in the United States, more commonly in older patients (>50 years old) though some subtypes (e.g., rhabdomyosarcoma, a sarcoma of the skeletal muscle) are more common in children and adolescents. Soft tissue sarcomas as a class are more common in companion animals, representing 15% of all subcutaneous cancers in dogs and 7 % in cats. With the exception of hemangiosarcomas, this class of tumors is locally aggressive but rarely metastasizes. Nevertheless, the soft tissue sarcomas of both humans and companion animals are only moderately responsive to chemotherapy.

Because they resemble human tumors of the same origin and are detected relatively late, providing greater tumor bulk for analysis, canine soft tissue sarcomas have served as models for optimizing therapeutic strategies. Protocols aimed at increasing local control, particularly those using adjuvant radiation, often coupled with hypothermia, have guided new treatment protocols for human patients. Interest in localized hyperthermia was stimulated by the observation that heat could increase the efficacy of radiation or chemotherapy. Local and whole body hyperthermia studies tested pharmacological approaches to inducing hyperthermia such as vasoactive drugs. Studies in dogs have also modeled the effect of hyperthermia on the pharmacokinetics of chemotherapeutic drugs and aided the development of biomarkers of hypoxia and prognostic imaging techniques. Soft tissue sarcomas in companion animals have also served as models for testing new chemotherapeutic formulations. For example, the efficacy of slow release cisplatin in a biodegradable polymer was tested in canine soft tissue sarcoma, and efficacy of liposome-encapsulated doxorubicin (Doxil) was tested in vaccine-associated feline sarcoma.

### Hemangiosarcoma

Hemangiosarcoma (HSA) is a tumor of the vascular endothelial cells characterized by rapid and extensive metastasis. It is rare in humans, accounting for less than 1% of all tumors, but accounts for 5-7% of all canine malignancies. Assuming a lifetime cancer risk for dogs in the range of 30-50% this cancer may affect 1.5-2.5 million of the estimated 72 million pet dogs in the United States. HSAs originate most often in the spleen, but can also form in the liver, right atrium of the heart, and skin. They tend to occur in middle aged dogs (> 6 years old), with higher prevalence in Bernese Mountain Dogs, Boxers, Flat Coated Retrievers, German Shepherds, Golden Retrievers, Portugese Water Dogs, and Skye Terriers; according to one survey the incidence of HSA in Golden Retreivers is almost 1 in 5. Canine HSAs appear comparable to angiosarcomas in humans, and because they occur with far greater frequency may prove an important surrogate for clinical testing. Chemotherapy, typically combinations of doxorubicin and cyclophosphamides +/- vincristine, are the most common therapeutic approach for HSA, but median survival times are only 145-180 days.

### Mammary Carcinoma

Breast cancer and canine mammary gland tumors have several epidemiological and physiological similarities. Breast cancer is the leading cause of cancer in North American women, accounting for nearly 30% of all cancer; the lifetime risk of breast cancer in American women is 12%. Mammary gland tumors (MGTs) account for 52% of all tumors in female dogs, and occur in 26% of all unsprayed dogs. There are significant genetic and histological similarities between breast cancer and MGT, but also key differences in gene expression and drug response that complicate efforts to translate therapeutic strategies between species. MGTs are hormone-dependent; 50-60% of these tumors express estrogen receptors or progesterone receptors, and ovariohysterectomy (spaying) reduces the risk of developing MGT to 0.5%. Human breast cancer is also hormone-dependent and often treated with drugs that affect estrogen or progesterone receptors, but the estrogen receptor antagonist tamoxifen does not have demonstrable anti-tumor activity in dogs. There are also similarities and differences on the genetic level. Expression of the oncogene c-erbB-2 correlates with a more aggressive malignant phenotype in human breast cancer. Similarly, c-erbB-2 is overexpressed in 74% of malignant canine mammary tumors, but in 0% of benign tumors. Mutations of the tumor-suppressor gene BRCA1/BRCA2 are associated with increased risk of human breast cancer. The expression and variants of BRCA1 are less documented in canine mammary gland tumors, though recent reports of splicing variants of BRCA1 in some MGTs and upregulation of BRCA2 and RAD51 (which interacts with BRCA1 and BRCA2) in metastases of MGTs point to the need for more extensive analysis of gene expression in these canine tumors. As with hormone treatment, the application of chemotherapeutic agents to the treatment of MGT is uncertain. According to some reviews, no chemotherapeutic agents have proven consistently effective in canine MGT, though a few partial responses to doxorubicin have been documented and cisplatin is sometimes recommended. Despite many similarities it remains unclear whether canine MGT is a relevant therapeutic model for human breast cancer. Additional studies of gene expression may identify common targets for human breast cancer and MGT and guide the application of human chemotherapies for treatment of the canine tumor.

### Melanoma

Skin cancer is the most common of all cancers in the United States. Although melanoma is a relatively uncommon form, accounting for less than 5% of skin cancer cases, it is responsible for 75% of skin cancer deaths. The rate of new cases was relatively stable over the past 8 years, with estimates of 68,720 new cases in 2009 resulting in over 8,650 deaths. According to a World Health Organization report, there are approximately 48,000 melanoma-related deaths worldwide per year. The overall risk of melanoma varies with ethnicity, ranging from 2% for Caucasians to 0.5% for Hispanics and 0.1% in African Americans. Current treatment options include surgical resection and chemotherapy (including single or combination treatments with dacarbazine, carmustine, cisplatin, tamoxifen, vinblastin, temozolomide, and paclitaxel). Melanoma is the fourth most common cancer in dogs, frequently occurring in the oral cavity but also originating in the digits, skin, and eye. Oral melanoma is reportedly most commonly observed in Dachshunds, Golden Retrievers, Poodles, and Scottish Terriers. As with advanced melanomas in humans, melanomas in dogs are generally resistant to chemotherapy and radiation, and aggressive metastasis is the primary cause of treatment failure and death.

Because canine and human melanomas share common features of physiology and response to treatment, clinical trials in dogs can provide an important translational bridge to new treatment strategies for human melanoma. Immunotherapy approaches have included autologous tumor cell vaccines (unmodified or transfected with immunostimulatory cytokines and/or melanosomal differentiation antigens), allogeneic tumor vaccines transfected with immunostimulatory cytokines (e.g. IL-2, GM-CSF), innate immune stimulants (e.g. L-MTP-PE), and DNA vaccine (e.g., plasmids encoding Fas ligand, IL-2, or GM-CSF). A randomized clinical trial of L-MTP-PE in canine melanoma showed an 80% long-term survival benefit in stage I melanoma, but no benefit in more advanced (stage II and III) melanoma. In a phase I clinical trial, vaccination with GM-CSF-transfected autologous melanoma cells induced localized inflammation and some histological evidence of tumor disruption. Other vaccine approaches have injected plasmid DNA directly into the melanoma. A phase I clinical trial of 9 dogs with stage II-IV advanced malignant melanoma injected DNA encoding the melanosomal differentiation antigen tyrosinase in attempt to induce cell mediate immunity against tumor cells expressing tyrosinase. This immunotherapy induced an antibody response in 33% of the treated dogs and extended the median survival time to 389 days, significantly longer than the 1-5 months survival conferred by conventional therapies.

### Non-Hodgkins Lymphoma

Non-Hodgkins lymphoma (NHL) is the sixth leading cause of cancer death, with an incidence rate of 3-4% in the United States, resulting in an estimated 66,000 new cases in the US in 2009, and a 5 year survival rate of 50-60% for patients treated with chemotherapy. Over 95% of new cases occur in adults, with an average age of onset of 60 years old. NHL is also relatively common in dogs; its incidence rate is 25/100,000, accounting for 5% of all malignancies and 83% of all hematopoietic malignancies. Approximately 70-80% of canine NHL cases are of B lymphocyte origin, while the rarer T cell lymphomas are associated with a significantly poorer prognosis. The highest prevalence of NHL occurs in German Shepherds, Boxers, Poodles, Basset Hounds, and Saint Bernards. Most canine cases resemble stages III-IV of human NHL, and in the absence of therapy disease progression is relatively rapid, resulting in death within 4-6 weeks after diagnosis. In addition to histological similarities, canine and human NHL share similar chemotherapeutic drug sensitivities, including responsiveness to doxorubicin, cyclophosphamide, and vinca alkaloids. As with human clinical practice, most current treatment protocols for canine NHL employ multiple, alternating combinations of drugs, resulting in reported response rates in the range of 86-91%.

With an incidence rate of 125/100,000, NHI is the most common cancer in cats, comprising nearly one third of all feline tumors. In contrast to canine NHL, a significant proportion of feline NHL is of T lymphocyte lineage, the result of transformation by a retrovirus, feline leukemia virus (FeLV). As with dogs, feline NHL is very chemoresponsive- sequential combination chemotherapy achieves remission rates of 60-70%. Based on their similarities with human tumors, both canine and feline NHL have served as surrogates for optimizing therapeutic approaches (*see* Examples).

### Bladder Cancer

Bladder cancer is the fourth most common cancer in men and the ninth most in women in the United States. The disparity in incidence, 50,000 men and 16,000 women annually, may be related to the major role of androgen receptors in the development of bladder cancers. The majority of bladder cancers are transitional cell carcinoma (90%), originating in the cells lining the inside of the bladder; the remaining 10% include squamous cell carcinoma, adenocarcinoma, sarcoma, and small cell carcinoma. Transitional cell carcinoma (TCC) is also the most common form of canine urinary bladder cancer, closely resembling invasive human TCC in histology, biologic behavior, and response to therapy. As with other cancers, there is variation in susceptibility related to breed; for example, Scottish Terriers have an 18-fold increased risk to develop TCC.

Current treatment options for bladder cancer patients include surgery, radiation, and chemotherapy. Canine TCC is responsive to these approaches as well and has been a useful model for development and optimization of novel therapeutics. Canine TCC shows modest response to platinum and anthracycline-based protocols, with objective response rates of ∼30% and MSTs of 4-8 months. Treatment with the cyclooxyenase inhibitor piroxicam results in an objective response rate of 18% which can be further improved by the addition of cisplatin, but at the cost of unacceptable nephrotoxicity. Canine TCC has proved a useful model for preclinical investigation of photodynamic therapy.

We describe the use of the platform technology for the identification of one or more biomarkers associated with cancer and in some cases, a gene expression profile, proteomic profile or metabolomic profile of cancers. The platform technology can be used for multiplexing biomarkers. In one non-limiting example, methylation patterns on gene chips can be used to study normal vs. abnormal methylation patterns for various cancers. Another non-limiting example is the use of magnetic arrays that can house multiple biomarkers (e.g., 15-18 biomarkers) and be detectable at low amounts (e.g., 1 pg/ml). Another non-limiting example is the use of aptamers where hundreds of biomarkers can be assessed simultaneously or nearly simultaneously.

In some cases of cancer, paraneoplastic syndrome is observed. In one aspect, paraneoplastic syndrome is a disease or symptom that is the consequence of the presence of cancer in the body, but is not due to the local presence of cancer cells. These phenomena can be mediated by humoral factors (by hormones or cytokines) excreted by tumor cells or by an immune response against the tumor. Sometimes the symptoms of paraneoplastic syndromes show even before the diagnosis of a malignancy. Paraneoplastic syndromes can be divided into 4 main categories: endocrine, neurological, mucocutaneous and hematological paraneoplastic syndromes. In another aspect, paraneoplastic syndromes can be a group of rare disorders that are triggered by an abnormal immune system response to a cancerous tumor or a "neoplasm." Without being bound by theory, in one aspect, paraneoplastic syndromes can happen when cancer-fighting antibodies or white blood cells (e.g., T cells) mistakenly attack normal cells in the nervous system. Accordingly, in one embodiment, the immune system is left intact so that paraneoplastic syndrome can be more effectively studied.

In another aspect of the invention, the use of companion animals with spontaneously occurring cancer allows for the study of cancer is a form that has not been induced to progress to a more severe state. In one embodiment, the cancer being studied is pre-metastatic. The use of anti-cancer drugs may cause inflammation which may cause the cancer to progress from pre-metastatic cancer to a metastatic cancer. By using an animal model of spontaneously occurring diseases, such as cancer, the cancer that is examined is not further induced to progress into a form that it would otherwise not have progressed absent the chemotherapeutic and/or radiation intervention.

In this manner, the immune system of the animal is kept in as close of the natural state as possible. This makes for more accurate studying of the biological or physiological state of the immune system and thus, allows for the generation of more meaningful scientific data. This scientific data can then be used for identification of anti-cancer therapeutic agents.

### Machine Readable Storage Media

The data generated by using the companion animal model system can be stored on machine readable media. Such data can include information about the biological responses, physiological parameters of responses to agents that are administered, antigen(s) which are identified, structure of agents that are administered, structure (including sequences, both nucleic acid and amino acid) of antigens or immunogens. This information can be stored on machine readable media and be further utilized to generated novel agents that have similar structure to known agents that have elicited a desirable immune response in the canine model system. In this manner, novel agents that have desirable biological effects are identified for potential use in treatment of humans.

We describe that machine readable storage media can be used for educational purposes, for example, instruction materials or manuals. We describe promoting collaborations between individuals, such as scientists, philanthropists, and veterinarians. Such collaboration can be fostered by dissemination of the data generated by use of companion animal model system of spontaneously occurring diseases. This dissemination can be accomplished by distribution of this data on tangible media, for example, a machine readable storage media.

We describe a machine-readable storage medium including a data storage material encoded with machine readable data which, when using a machine programmed with instructions for using said data, displays a graphical three-dimensional representation of any of the molecule or molecular complexes of this invention that have been described above. In a preferred embodiment, the machine-readable data storage medium includes a data storage material encoded with machine readable data which, when using a machine programmed with instructions for using said data, displays a graphical three-dimensional representation of a molecule or molecular complex.

For example, a system for reading a data storage medium may include a computer including a central processing unit ("CPU"), a working memory which may be, e.g., RAM (random access memory) or "core" memory, mass storage memory (such as one or more disk drives or CD-ROM drives), one or more display devices (e.g., cathode-ray tube ("CRT") displays, light emitting diode ("LED") displays, liquid crystal displays ("LCDs"), electroluminescent displays, vacuum fluorescent displays, field emission displays ("FEDs"), plasma displays, projection panels, etc.), one or more user input devices (e.g., keyboards, microphones, mice, touch screens, etc.), one or more input lines, and one or more output lines, all of which are interconnected by a conventional bidirectional system bus. The system may be a stand-alone computer, or may be networked (e.g., through local area networks, wide area networks, intranets, extranets, or the internet) to other systems (e.g., computers, hosts, servers, etc.). The system may also include additional computer controlled devices such as consumer electronics and appliances. This allows for collaborative efforts to be pooled for better results.

Input hardware may be coupled to the computer by input lines and may be implemented in a variety of ways. Machine-readable data of this invention may be inputted via the use of a modem or modems connected by a telephone line or dedicated data line. Alternatively or additionally, the input hardware may include CD-ROM drives or disk drives. In conjunction with a display terminal, a keyboard may also be used as an input device.

Output hardware may be coupled to the computer by output lines and may similarly be implemented by conventional devices. By way of example, the output hardware may include a display device for displaying a graphical representation of an active site of this invention using a program such as QUANTA. Output hardware might also include a printer, so that hard copy output may be produced, or a disk drive, to store system output for later use.

In operation, a CPU coordinates the use of the various input and output devices, coordinates data accesses from mass storage devices, accesses to and from working memory, and determines the sequence of data processing steps. A number of programs may be used to process the machine-readable data of this invention. Such programs are discussed in reference to the computational methods of drug discovery as described herein. References to components of the hardware system are included as appropriate throughout the following description of the data storage medium.

Machine-readable storage devices include, but are not limited to, magnetic devices, electrical devices, optical devices, and combinations thereof. Examples of such data storage devices include, but are not limited to, hard disk devices, CD devices, digital video disk devices, floppy disk devices, removable hard disk devices, magneto-optic disk devices, magnetic tape devices, flash memory devices, bubble memory devices, holographic storage devices, and any other mass storage peripheral device. It should be understood that these storage devices include necessary hardware (e.g., drives, controllers, power supplies, etc.) as well as any necessary media (e.g., disks, flash cards, etc.) to enable the storage of data.

The following examples are provided for illustrative purposes only and are not meant to limit the scope of the invention in any manner.

### EXAMPLES

### Example 1 Preparation of Delivery Vehicles For Use in the Animal Model of Spontaneously Occurring Diseases

Delivery vehicles that selectively seek out a cancer cell instead of a normal cell is prepared by using molecular or physical property or biomarker properties that allows for selective targeting. In this example, the delivery vehicle is a liposome, liposome-like particle or nanoparticle. The liposomes can be charged (e.g, cationic) or non-charged. These liposomes, liposome-like particles or nanoparticles are made both with and without receptor or ligands or biomarkers.

Liposomes, liposome-like particles or nanoparticles are also made which carries one or more oncolytic viruses (for example, any of the oncolytic viruses discussed in "Viral Therapy of Cancer," Harrington, Vile and Pandha, co-editors, Wiley Publishing, 2008). Other liposomes, liposome-like particles or nanoparticles are made which carry prodrugs and RNAi targets, with or without immune cells or chemotactic agents or immune modulators.

The prodrugs are incorporated into the liposomes, liposome-like particles or nanoparticles and used for testing in the animals with spontaneously occurring diseases. Non-limiting examples of products are cancer therapeutics and other drugs for cancer. Similarly, RNAi targets are incorporated into the liposomes, liposome-like particles or nanoparticles. For RNAi targets, proto-oncogenes and oncogenes, the RNAi(s) serves to turn off, block, or reduce the activation of proto-oncogenes and oncogenes. For tumor suppressors, the RNAi(s) serves as an agonist to turn on or increase the activity of the tumor suppressors.

The liposomes, liposome-like particles or nanoparticles of this example are also packaged with immune modulators, which include cytokines, chemokines, exosomes (small particles secreted by various immune cells, such as mastocytes, T and B lymphocytes, dendritic cells, platelets) or immune factors that promote differentiation/maturation/clonal expansion of immune cells (e.g., CTLA-4). Immune modulators that are incorporated into the liposomes, liposome-like particles or nanoparticles of this example can also target the immunosuppressor cells (e.g., T regulatory cells or MDSCs) to potentiate cancer immunotherapy.

Liposomes, liposome-like particles or nanoparticles of this example are also packaged with factors that affect epigenetics, for example, methylation, prenylation, acetylation and de-acetylation (e.g., histone acetyltransferases (HATs) and histone deaceytlases (HDACs)), chromatin modifications, X-inactivation, and imprinting.

Liposomes, liposome-like particles or nanoparticles of this example are engineered with various molecular properties that are helpful to make these delivery vehicles more effective. Cancer antigens and other types of biomarkers (metabolic markers) are examples of molecular properties. See Example 3 for more details on cancer antigens. Another molecular property is ligand binding. Pre-metastatic niches are targeted by using the appropriate ligand for binding. Similarly, metastatic niches are also targeted. Some markers are used for certain types of cancer. For example, the Axl receptor is used to target pancreatic cancer since it is expressed >50% in metastatic pancreatic cancer. Cancer Biol Ther. 8(7):618-26 (2009). An example of a metabolic marker is formerly N-linked glycopeptides that change in abundance upon cAMP treatment in glioblastomas. Proteomics 9(3):535-49 (2009).

Liposomes, liposome-like particles or nanoparticles of this example are also engineered by using various physical properties. Tumor tissues have a different physical gradient than non-tumor tissue. Pressure gradient of tumor versus non-tumor tissues are measured by standard techniques known to those of skill in the art. The liposomes, liposome-like particles or nanoparticles as described above are made for preferential targeting to tumors and tumor-bearing regions of the body by following the pressure gradient of the tumors.

### Example 2 Timing and Dosing of Delivery of Agent(s)

In this example, a cohort of a homogeneous or heterogeneous canine population is used as own control. The dosing of one or more agents under investigation is about one week in between doses. The order of delivery between cancer therapy and immune modulator is varied and the biological responses are measured and/or monitored. In one group of canines, cancer therapy is administered first and then the immune modulator(s). In another group of canines, the immune modulator(s) is administered first and then cancer therapy.

In another group of animals, the order of delivery of immune modulators with or without chemotaxis agent is switched and biological responses are then measured.

### Example 3 Canine and Cancer Antigen/Biomarkers

Multiple cancers antigens and/or biomarkers are used for translational studies in various combinations with each other. For osteosarcoma, cancer antigens and/or biomarkers that are examined include but are not limited to: the antigen that is bound by monoclonal antibodies TP-1 and TP-3 (which detect an antigen expressed on the cells of human osteosarcoma), *erbB-2* (human epidermal growth factor receptor 2/neu) proto-oncogene, vimentin, osteopontin, PCNA, p53, MMP-2 and MMP-9.

For lymphoma (e.g., non-Hodgkin lymphoma), antigens and/or biomarkers that are examined include but are not limited to: CD3 antigen (J Vet Diagn Invest 5:616-620, 1993), T200 (homologue of the lymphocyte differentiation antigen)(Can J VetRes. 51(1): 89-94, 1987), and the antigen that is bound by canine lymphoma monoclonal antibody 231 (Cancer Therapy, Vol 7, 59-62, 2009).

For hemangiosarcoma, antigens and/or biomarkers that are examined include but are not limited to: c-kit, CD34, CD133, CD45 (Exp Hematol., 34(7):870-8, 2006), factor VIII-related antigen, ICAM-1, atß3 integrin (Research in Veterinary Science, 81(1): 76-86, 2006), VEGF receptors 1 and 2, CD31, CD146, and avß3 integrin (Neoplasia, 6(2): 106-116, 2004).

For mammary cancer, antigens and/or biomarkers that are examined include but are not limited to: Receptor-binding cancer antigen expressed on SiSo cells (RCAS1) (Journal of Veterinary Medical Science, 6 (6): 651-658, 2004), Sialyl Lewis X and T/Tn (Vet Pathol 46:222-226, (2009).

For testicular cancer, antigens and/or biomarkers that are examined include but are not limited to: proliferating cell nuclear antigen (PCNA)(Journal of Comparative Pathology, 113(4): 301-313, 1995), GATA-4 (transcription factor expressed in Sertoli cells and less commonly in Leydig (interstitial) cells) (Veterirtary Pathology, doi:10.1354/vp.08-VP-0287-R-BC, 2009), inhibin-alpha and vimentin (J. Vet. Sci., 10(1), 1-7, 2009).

For mast cell cancer, antigens and/or biomarkers that are examined include but are not limited to: CD 117 *(*BMC Vet Res. 3:19, 2007), chromosome nucleolar organizer regions stained with silver (AgNORs), and anti-proliferating cell nuclear antigen (PCNA)(Veterinary Pathology, Vol 31, Issue 6, 637-647, 1994).

For bladder cancer, antigens and/or biomarkers that are examined include but are not limited to: V-TBA, or urinary tumor bladder antigen (Am J Vet Res. 64(8):1017-20, 2003) and basic fibroblast growth factor (bFGF).

For prostate cancer, antigens and/or biomarkers that are examined include but are not limited to: prostatic phosphatic acid antigen, prostate specific antigen (PSA), prostate specific membrane antigen (PMSA), and downregulation of epithelial Na, K-ATPase expression (Cancer Cell Int. 3:8, 2003).

For melanoma, antigens and/or biomarkers that are examined include but are not limited to: canine melanoma antigen recognized by the murine monoclonal antibody IBF9 (Am J Vet Res. 58(1): 46-52, 1997), S100, human melanosome specific antigens (HMSA) 1 and 5, neurone specific enolase (NSE), vimentin and IBF-9 (http://www.vetscite.org/publish/articles/000038/index.html).

For leukemia, antigens and/or biomarkers that are examined include but are not limited to: rearrangement of TCR Vβ genes (e.g., detection of seven distinct canine TCR Vβ genes)(Veterinary Immunology and Immunopathology, Vol. 69, Issues 2-4, Pages 113-119, 1999)

For lung carcinoma, antigens and/or biomarkers that are examined include but are not limited to: proliferating cell nuclear antigen (PCNA) and Ki-67 (MIB1) proteins (Journal of Comparative Pathology, 120(4): 321-332, 1999).

### Example 6 Myeloid Suppressor Cell Depletion to Augment Tumor Vaccine Responses in a Canine Model of Non-Hodgkin Lymphoma

This example contains references to publications by use of numbers which correspond to the list of publications at the end of the example. The overall goal of this example is to develop more effective therapeutic cancer vaccines by utilizing MSC depletion to augment immune responses to existing cancer vaccines. The success rate of current tumor vaccines remains low despite a tremendous amount of effort directed to vaccine design. The relative ineffectiveness of current cancer vaccines may stem in part from the immunosuppressive properties of myeloid suppressor cells (MSC), which serve to potently suppress not only antitumor immunity, but may also suppress immune responses to vaccines in general. Preliminary studies indicate that elimination of MSC using liposomal clodronate (LC) can trigger spontaneous T cell-mediated antitumor immunity. Moreover, preliminary studies also indicate that MSC depletion can increase immune responses to vaccines in animals without tumors. Therefore, this example details how MSC depletion affects the generation of antitumor immunity following tumor vaccination, using both mouse and dog tumor models. Next, using a spontaneous canine model of Non-Hodgkin Lymphoma (NHL), the question of whether the combined MSC depletion/tumor vaccination approach is more effective in reducing minimal residual tumor burden than tumor vaccination alone is examined.

Aim: To determine using mouse tumor models the optimal timing of MSC depletion for augmenting immune responses to tumor vaccination. The non-binding hypothesis is that depletion of MSC shortly after vaccination will significantly enhance T cell responses to vaccination and trigger significantly enhanced antitumor activity.

### Background and Rationale for Cancer vaccines and NHL

Non-Hodgkin lymphoma (NHL) is an important tumor of humans that has been considered a prime target for vaccine immunotherapy because the tumor cells each express a unique tumor antigen (i.e., the idiotypic surface immunoglobulin molecule). Most forms of NHL are relatively refractory to treatment with chemotherapy and affected patients typically have short survival times. Therefore, a number of tumor vaccine approaches for NHL have been devised (1-5). Most NHL vaccines have utilized the idiotypic antigen receptor as the target antigen for immunization. Numerous vaccine studies have been conducted in NHL patients and three NHL studies have advanced to the point of completing phase III clinical trials (5, 6). Unfortunately, despite encouraging preliminary results, each of the phase III trials completed thus far has failed to meet the original study endpoints (5). The reasons for the vaccine trial failures are not clear, but may be related to vaccine design, insufficient vaccine potency, or patient inclusion criteria.

Despite a lack of major clinical successes, significant progress has been made in the design and implementation of cancer vaccines over the past two decades. However, there have still been few human cancer vaccines that have advanced beyond phase I trials. Thus, it is apparent that incremental improvements in vaccine design may not be sufficient to overcome the considerable hurdles that cancer vaccines face. Therefore, the focus of research in cancer immunotherapy has now begun to shift towards a better understanding of the role of the tumor microenvironment in regulating tumor immunity. One new strategy to emerge from this refocusing is the idea that modifying or circumventing immune regulatory and inhibitory mechanisms could be used to improve the effectiveness of existing tumor vaccines.

***Myeloid suppressor cells (MSC) inhibit antitumor immunity.*** A number of recent studies have begun to more fully define the key role that immature myeloid cells play in suppressing tumor immunity (7-11). This poorly defined population of myeloid cells are referred to collectively as myeloid suppressor cells (MSC). Recently, the MSC population in animals with cancer has been shown to consist of a mixture of immature monocytes and neutrophils(12). Despite their differing lineage, both monocytic and neutrophilic MSC have been shown to suppress T cell and NK cell function, albeit by different mechanisms. Suppression of T cells and NK cells is mediated by a number of mechanims, including production of reactive nitrogen species, reactive oxygen species, and surface expression of TGF-β, and arginase production. In many cases, inhibition by MSC requires direct or very close contact with T cells. The end result is that T cells and NK cells in the vicinity of MSC are rendered functionally incapable of cytotoxicity, proliferation, and cytokine production. The generation of MSC from the bone marrow is regulated by cytokines and growth factors produced by tumor cells themselves, or produced in response to tumor-associated inflammation. Following release from the bone marrow, MSC distribute to the spleen, bone marrow, draining lymph nodes, and tumor tissues.

Myeloid suppressor cells are not only generated in response to cancer, but are also elicited by a variety of inflammatory stimuli. For example, expanded numbers of MSC are present in individuals with sepsis, chronic infections (viral, fungal), and chronic inflammatory diseases (12). Thus, it appears that MSC likely have evolved to serve as negative modulators of both acute and chronic inflammation (7, 13). Viewed therefore as regulators of inflammation, without being bound by theory, MSC may also serve to dampen immune responses to vaccines, especially vaccines that elicit significant inflammation. Such a response would be particularly pronounced in individuals with cancer, since they would already possess greatly expanded numbers of MSC(14). In fact, evidence for just such an MSC response to tumor vaccination has been reported in melanoma patients vaccinated with a GM-CSF transduced melanoma vaccine (15, 16). If MSC do in fact inhibit tumor vaccine responses, then eliminating MSC or blocking their effects may help boost effective T cell immune responses to vaccination in patients with cancer. Experimental evidence in favor of this idea comes from studies of all-trans retinoic acid (ATRA) induced differentiation of MSC, which drives the cells to a mature into macrophages or neutrophils and reverses their immunosuppressive properties. When tumor-bearing animals or humans were treated with ATRA, spontaneous anti-tumor immunity was improved and vaccine responses were significantly enhanced (17-19). Similar enhancement of tumor vaccine responses was also reported when ROS production by MSC was inhibited using nitroaspirin (20).

One question is whether MSC depletion can restore tumor immunity and improve the effectiveness of tumor vaccines. Based on the preceding information, without being bound by theory, elimination of MSC may improve tumor vaccine responses. At present, the only two realistic options for eliminating MSC *in vivo* are to use antibody-mediated depletion or to use liposomal clodronate. Antibody mediated depletion of MSC has shown some effectiveness *in vitro* and *in vivo,* but is not currently considered feasible because a cell surface marker specific for MSC has not been identified (21). However, non-specific depletion of CD11b⁺/Gr-1⁺ cells with antibodies results in widespread depletion of macrophages, monocytes, and neutrophils and increases the risk of immunosuppression. Liposomal clodronate (LC) has been used extensively in the past to deplete macrophages and monocytes in mice for a variety of immunological investigations (22-26). When the bisphosphonate drug clodronate is encapsulated within neutral liposomes, the liposomes are taken up efficiently by phagocytic myeloid cells (macrophages, monocytes, MSC), followed by intracellular release of clodronate and rapid induction of macrophage apoptosis through competition for ATP binding (27, 28). Because LC does not deplete neutrophils, the risks of significant immunosuppression are considerably reduced.

More recently, LC treatment has also demonstrated antitumor activity in rodent tumor models, though in these studies the antitumor effects of LC treatment have been attributed to the effects of depletion of tumor-associated macrophages (TAM) and inhibition of tumor angiogenesis (29-31). The use of LC as a macrophage depleting agent in mouse models and in dogs with autoimmune disease has been investigated (32, 33). In addition to depletion of macrophages, systemic (intravenous) administration of LC also induced significant MSC depletion, which was associated with significant anti-tumor activity in mice and in dogs (34).

However, the query was whether LC treatment might be mediating antitumor activity through induction of systemic immune effects, rather than by local depletion of TAM in tumor tissues. Indeed, the antitumor activity elicited by LC treatment was due to spontaneous, systemic activation of antitumor immunity, rather than by depletion of TAM or inhibition of tumor angiogenesis. Thus, without being bound by theory, MSC depletion using LC could, if administered in the proper sequence relative to vaccination, also significantly augment the effectiveness of tumor vaccines. In fact, experiments combining LC treatment and vaccination against model antigens suggest that just such an effect occurs. Therefore, without being bound by theory, MSC depletion using LC improves the effectiveness of NHL tumor vaccines. This example investigates this hypothesis first in mouse tumor models and to then conduct proof-of-principle experiments in a canine model of NHL.

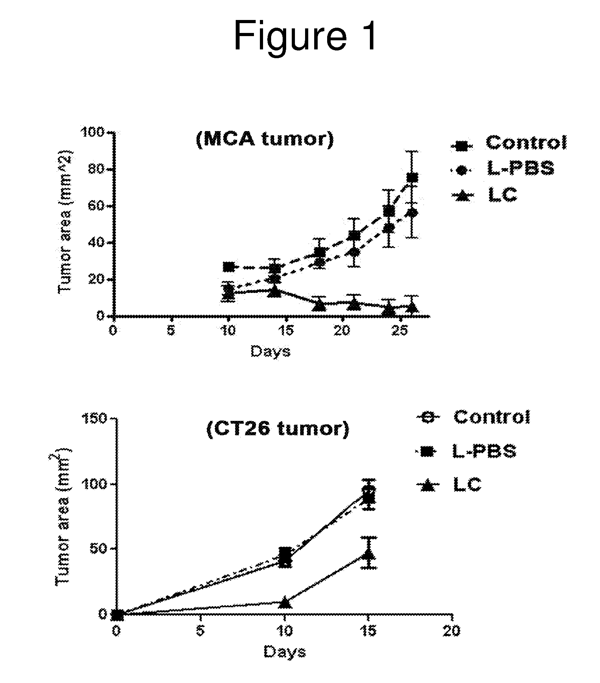

Results: Past studies on the antitumor activity elicited by systemic administration of LC have focused in part on determining how to optimize LC delivery to generate maximal antitumor activity, on assessing the spectrum of tumor types that are susceptible to LC-induced antitumor activity, and on defining the mechanism(s) by which LC generates antitumor activity. Intravenous administration of LC elicits significant inhibition of growth of established tumors in mouse models. For example, once weekly i.v. administration of 200 ul LC to C57B1/6 mice with established s.c. MCA-205 (sarcoma) tumors produced significant inhibition of tumor growth (Figure 1). Importantly, administration of control PBS containing liposomes (L-PBS) did not elicit antitumor activity. Similar antitumor activity was also generated in BALB/c mice with CT-26 (colon carcinoma) tumors. Significant antitumor activity was also observed in mice with B16 (melanoma) and 4T1 (breast carcinoma) tumors. Thus, LC administration inhibits tumor growth in a tumor type and mouse strain independent fashion.

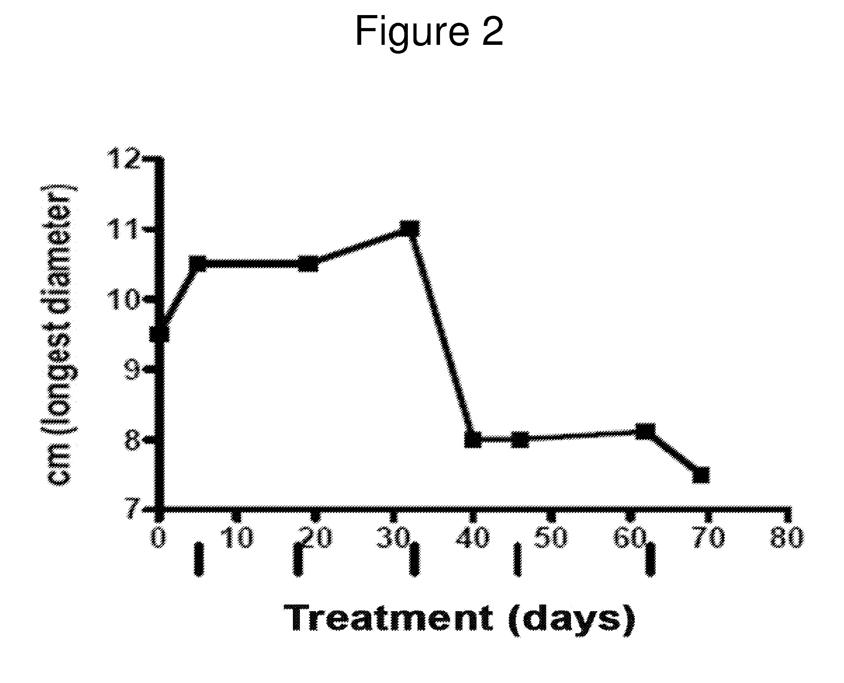

Studies in dogs have also demonstrated that LC has antitumor activity. For example, twice monthly i.v. administration of LC to dogs with soft tissue sarcoma (STS) or malignant histiocytosis (MH) elicits tumor regression in approximately 50% of treated patients. As shown in Figure 2, a dog with STS treated with a series of treatments with LC alone experienced significant spontaneous tumor regression beginning after the third LC administration. Treatment responses have also been observed in dogs with MH treated with LC (34). Importantly, treatment with LC was well-tolerated by dogs, even those with advanced cancer, and the only notable side-effect has been transient fever, which has interestingly only been observed in dogs with MH. Thus, LC is also an effective and well-tolerated antitumor agent in dogs with cancer.

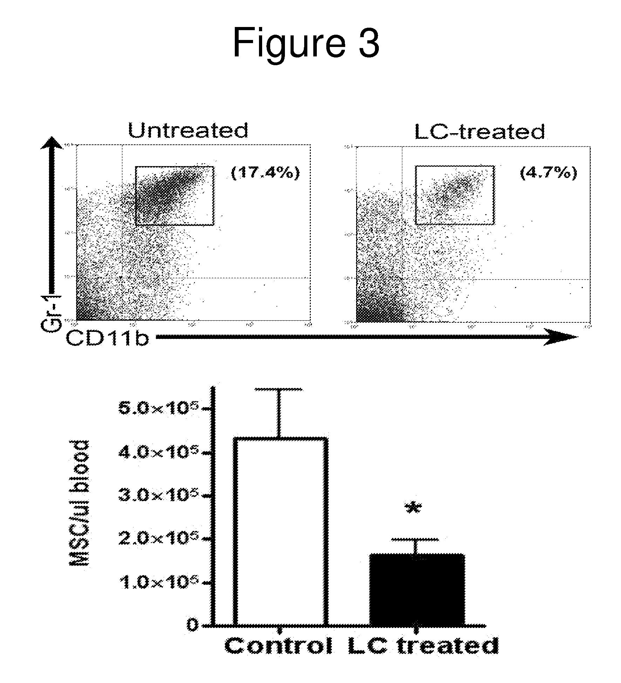

Studies to elucidate the immunological mechanisms by which LC treatment may induce spontaneous antitumor activity have been done. Since LC is known from prior studies to deplete phagocytic cells, whether LC treatment could deplete myeloid suppressor cells (MSC), particularly monocytic MSC(35) was investigated. Twenty-four hours after i.v. administration of LC in tumor-bearing mice, CD11b⁺/Gr-1⁺ MSC were enumerated in spleen, blood, and tumor tissues (Figure 3). Significant MSC depletion occurred in blood (Figure 3), spleen, and tumor tissues, and that most of the depleted cells were monocytic. In addition, there was significant depletion of TAM and inhibition of tumor angiogenesis in LC-treated mice. Thus, systemic administration of LC elicited significant depletion of multiple different populations of phagocytic myeloid cells, including MSC, in animals with cancer.

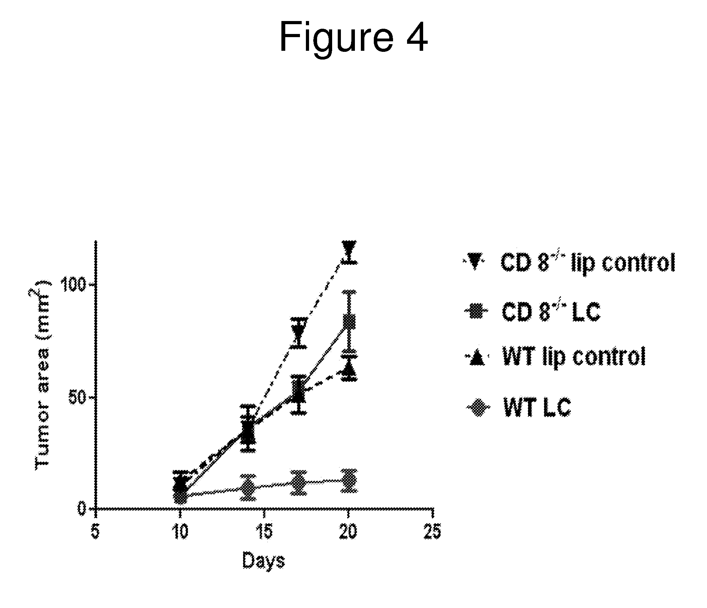

Based on the fact that i.v. administration of LC resulted in systemic depletion of MSC, the next step was to investigate whether the antitumor effects of LC treatment were mediated by local effects (i.e., depletion of TAM) or by systemic immunological effects. To address this question, tumor-bearing mice lacking T cells (RAG2^{-/-} mice) were treated with LC and compared MCA tumor growth rates with wild type C57B1/6 mice treated with LC. The antitumor effect of LC treatment was almost completely abrogated in RAG2^{-/-} mice, which suggested that the antitumor activity of LC was largely mediated by T cells. Therefore, to determine which T cell subset mediated the antitumor activity of LC, the tumor experiment in CD8^{-/-} mice and CD4^{-/-} mice was repeated. The antitumor activity of LC was almost completely eliminated in CD8^{-/-} mice (Figure 4), whereas the activity of LC was only partially inhibited in CD4^{-/-} mice. Controls also included mice treated with PBS containing liposomes (lip control). Therefore, the antitumor activity elicited by i.v. administration of LC was mediated by systemic activation of CD8 T cell anti-tumor immunity, rather than by local effects on TAM or tumor angiogenesis. These results are important because they suggest that MSC depletion and activation of systemic immunity is likely the primary mechanism by which LC generates antitumor activity.

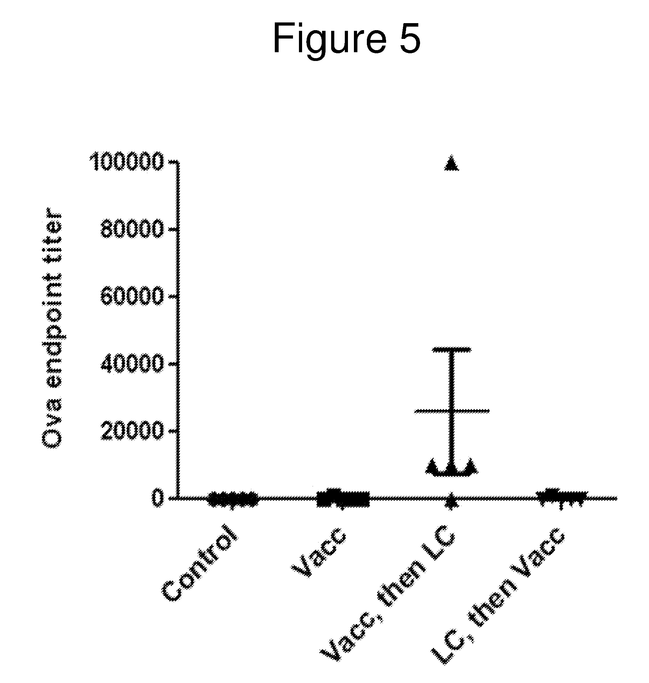

The preceding experiments, in which LC-mediated depletion of MSC was able to generate spontaneous CD8 T cell-mediated antitumor activity, also suggested that MSC depletion might be capable of enhancing vaccine responses. To address this question, mice were vaccinated s.c. using a CLDC-adjuvanted vaccine (36) containing ovalbumin as a model antigen and asked whether MSC depletion using LC could enhance vaccine responses, using humoral immune responses as the readout (Figure 5). Mice were vaccinated once with the ova/CLDC vaccine alone, or with ova/CLDC plus LC treatment 3 days prior to immunization (LC, then Vacc), or ova/CLDC plus LC treatment 3 days after immunization (Vacc, then LC). Blood was collected and IgG responses to ova determined by ELISA. The mice vaccinated with ova/CLDC and treated with LC 3 days after immunization developed significantly higher antibody responses than mice vaccinated with ova/CLDC alone or ova/CLDC plus LC 3 days before immunization. Thus, these data suggest that in fact MSC depletion can enhance immune responses to vaccination when administered in the proper sequence relative to the vaccine. Moreover, it should also be noted that this experiment was conducted in non-tumor bearing mice, while the vaccine enhancing effect would be expected to be more pronounced in tumor-bearing animals with much larger populations of MSC.

### Experimental Design

Aim: Determine how the timing of MSC depletion affects vaccine-induced T cell responses.

Though LC is effective in depleting MSC, administration of LC also results in depletion of other relevant myeloid cells, including macrophages and DC. Thus, it is possible that LC administration could inhibit or augment vaccine responses, depending on which cells were depleted and when they were depleted relative to vaccination. Therefore, mouse immunization models are used to determine the effect of timing of systemic LC administration on cellular and humoral immune responses to vaccination with CLDC adjuvanted vaccines. Initial experiments use a model antigen, since the readouts for these experiments are very robust and reproducible. Once the optimal timing of administration is identified, the relevance of these findings in two mouse cancer models are confirmed. The B16 melanoma model was selected because CD8 T cell responses can be tracked using tetramers, while the A20 lymphoma model was selected because of the close similarity with the dog NHL model. In addition, A20 cell lines that have been transfected with the HA antigen are used, which allow more accurate assessment of CD4 T cell responses.

### Experimental Approach:

Aim: to determine the optimal timing of MSC depletion to increase T cell and antibody responses to immunization with a nominal antigen or with a tumor antigen. These experiments are designed to 1) determine whether combined MSC depletion and vaccination enhances immune responses in normal and tumor-bearing mice; 2) identify the optimal timing of MSC depletion relative to vaccination to maximize immune responses; and 3) to assess the effects of combined MSC depletion and immunization on antitumor immunity in two mouse tumor models.

| **Table 1. Timing of depletion** | | |
|---|---|---|
| **Group** | **Vaccinate** | **MSC deplete** |
| 1 | - | - |
| 2 | + | - |
| 3 | - | + |
| 4 | + | day -7 |
| 5 | + | day -3 |
| 6 | + | day -1 |
| 7 | + | concurrent |
| 8 | + | day +1 |
| 9 | + | day +3 |

*Determine optimal timing of MSC depletion relative to vaccination to elicit maximal T cell responses.* These experiments are designed to determine the optimal timing of MSC depletion using LC treatment for generating maximal T cell responses. In the first experiments, normal C57B1/6 mice (n = 5 per group) re be vaccinated with Ova, using a potent cationic liposome-nucleic acid (CLDC) adjuvant developed as in the referenced publication (36). The 10 experimental groups of animals to be evaluated are described in Table 1. Mice are vaccinated s.c. with 5 ug Ova in CLDC adjuvant. Depletion of MSC is accomplished using a single injection of 200 ul liposomal clodronate (LC) administered i.v. Mice are euthanized 7 days after vaccination and lymphoid tissues and serum collected. Read-outs include assessment of CD8 responses by flow cytometry (K^{b}-ova tetramers), CD4 responses (cytokine release and proliferation assays), and humoral responses (serum antibodies to Ova are quantitated by ELISA).

*Statistical analysis of data*. Immune responses in treated mice are compared to untreated control mice, using non-parametric ANOVA (Kruskal-Wallis), followed by Dunn's multiple means comparison test. Similar analyses are also done for data below. Statistical analysis is done using commercial software (Prism5, GaphPad, San Diego, CA) and significance is defined as p < 0.05.

Treatment with LC either 1 day or 3 days after immunization generates optimal immune responses, which is reflected by increased numbers of Ova-specific CD8 T cells, greater IFN-γ production, and higher antibody titers. These assays are routinely done in the laboratory. The results allow clear identification of the optimal timing of MSC depletion to enhance vaccine responses. If readouts are not clear after a single immunization, then the experiment is repeated using a boost immunization administered 2 weeks after the first immunization to increase the numbers of antigen specific T cells.

*Assess the effects of MSC depletion on immune responses and antitumor activity following vaccination against tumor antigens.* These experiments are designed to determine whether MSC depletion can augment T cell responses against tumor antigens in mice with established tumors, using two different tumor models. In the first model, C57B1/6 mice with B16 melanomas are used, because a well-defined tumor antigen (trp2) has been identified in this model which allows accurate quantitation of CD8 T cell responses using tetramer reagents. Using the optimal MSC depletion schedule determined above, mice (n = 5 per group) with established cutaneous B16 tumors are vaccinated s.c. with 5 ug trp2 peptide in CLDC adjuvant, then boosted 7 days later. Treatment groups include unvaccinated control mice, vaccinated only mice, LC only treated mice, and mice treated with vaccination plus LC treatment. Numbers of trp2-specific CD8 T cells in blood, spleen, and LNs is assessed 5 days after the boost, using flow cytometry and Kb-trp2 tetramers. These experiments are repeated in another group of mice to assess the effects of combined MSC depletion/vaccination on tumor growth responses. In these studies, tumor growth rates are assessed by means of 3 times/week measurement of tumor diameter. In addition, the overall survival times of treated and control mice are evaluated.

In a second tumor model, immune responses to vaccination in BALB/c mice with A20-HA lymphomas are evaluated. In this model, the tumor has been engineered to express the influenza HA antigen to facilitate measurement of T cell responses. Two different vaccines are evaluated: 1) paraformaldehyde fixed A20-HA cells (1 X 10⁶ inactivated A20 cells per vaccine, admixed with CLDC adjuvant) or 2) HA antigen vaccine, 5 ug rHA in CLDC adjuvant. Mice (n = 5 per group) with established cutaneous A20 tumors are vaccinated s.c. with either autologous A20-HA tumor cells in CLDC adjuvant, or with rHA in CLDC adjuvant, then boosted 7 days later. Treatment groups include unvaccinated control mice, vaccinated only mice, LC only treated mice, and mice treated with vaccination plus LC treatment. Immune responses to be assessed include measurement of cytokine responses to vaccination (cytokine release following *in vitro* restimulation of spleen or LN cells with fixed tumor cells or with HA antigen), proliferative responses (proliferation of spleen or LN cells following *in vitro* restimulation for 96 hours with fixed A20 tumor cells or with rHA antigen) and assessment of *in vivo* CTL activity (*in vivo* killing of adoptively transferred, CFSE-labeled A20 tumor cells; described previously (36). The experiments are repeated in another group of mice to assess the effects of combined MSC depletion/vaccination on tumor responses. In these studies, tumor growth rates of cutaneously implanted A20 are assessed by means of 3 times per week measurement of tumor diameter. In addition, the overall survival times of treated and control mice is determined.

Combined MSC depletion/vaccination protocol induces a significant increase in the number of trp2-specific CD8 T cells in the B16 tumor model, compared to vaccination alone or MSC depletion alone. If an additive effect of the two treatments is not observed, the experiment is repeated using twice weekly LC administration, in case the numbers of MSC in tumor-bearing mice are still sufficient to inhibit vaccine responses. If the magnitude of trp2-specific CD8 T cell responses is too low to measure directly *ex vivo,* then the cells for 4-5 days are cultured *in vitro* in the presence of IL-2 and specific peptide to expand numbers of T cells before the tetramer assay is done. The increase in numbers of trp2 specific T cells correlates with a significant reduction in tumor growth rate and increased overall survival times in mice receiving combined MSC depletion/vaccination therapy. The role of CD8 T cells in the antitumor immune response is confirmed using CD8^{-/-} mice, or by antibody mediated depletion of CD8 T cells following immunization.

In the A20-HA model, T cell cytokine release and CTL activity is increased in mice that receive the combination MSC depletion/vaccination therapy. By utilizing both whole tumor cell and HA vaccination and immune assays, interpretable data is generated. Tumor growth rates are significantly slowed and survival improved in mice vaccinated with autologous tumor cells plus MSC depletion. Vaccination with the autologous tumor vaccine are most likely to be more effective than vaccination with the HA antigen alone due to the greater complexity and number of potential antigens on fixed tumor cells.

Aim: To determine whether tumor vaccination combined with MSC depletion significantly reduces residual tumor burden in a canine model of Non-Hodgkin lymphoma.

Rationale. Experiments in mouse tumor models are useful for optimizing the timing of vaccine and LC administration to maximize cellular immunity, and also for assessing antitumor activity. However, the limitations of mouse tumor models in predicting outcomes in human cancer studies are well-known. Therefore, the best available spontaneous NHL tumor model, dogs with B cell lymphoma, is used. This model has been used in the past to assess the effectiveness of an autologous lymphoma vaccine prepared using CM-CSF transfected tumor cells. The approach of vaccinating dogs with whole, fixed autologous tumor cells may not entirely analogous to human NHL vaccines, which usually consist of recombinant idiotypic Ig molecules however, constructing such a vaccine is highly difficult in the dog tumor model. Immunizing with paraformaldehyde fixed tumor cells, which preserve the surface Ig molecules, generates relevant vaccine responses. Using a conservative study design with 3 treatment groups of dogs, the determination of whether LC treatment can significantly augment NHL tumor vaccine responses is done. Moreover, use of changes in minimal residual disease burden (MRD) following vaccination as the primary endpoint for the study (rather than DFI or OST) allows study endpoints to be achieved much more quickly and with greater potential accuracy. This type of data is also highly relevant to the evaluation of MSC depletion therapy with LC as a strategy for use with human NHL vaccines.

*Trial design.* These studies are designed as a proof-of-principal study in dogs with B cell lymphoma, the canine equivalent of NHL in humans. The primary goal of this study is to determine whether vaccination plus MSC depletion generates a greater reduction in residual tumor burden (circulating tumor DNA detectable by qRT-PCR in the bloodstream (37) than vaccination alone or MSC depletion alone. Based in part on studies in mice, group sizes of 8 dogs each should allow the determination of a significant treatment difference, based on a 30% reduction in MRD in vaccinated/MSC depleted dogs compared to dogs that are vaccinated alone or treated with LC alone, with a power of 80% (PS Power and Sample Size calculation software). Therefore, 24 dogs with histologically confirmed B cell lymphoma are enrolled in a randomized clinical trial. Each dog is treated with conventional chemotherapy (doxorubicin plus Lasparaginase) for 10 weeks to achieve complete macroscopically visible tumor remission, at which time dogs are randomized to treatment group 1 (vaccine alone); treatment group 2 (LC treatment alone); or treatment group 3 (vaccine plus LC treatment). Group 1 and 3 dogs are vaccinated once every 2 weeks for 5 total immunizations, using autologous lymphoma cells (1 x 10⁷ paraformaldehyde-fixed cells per vaccination, administered s.c. in 2 ml CLDC adjuvant). Group 2 dogs receive a series of 5 infusions of LC (0.5 ml/kg) once every 2 weeks. Group 3 dogs are vaccinated and treated with LC, using the optimal timing of LC administration relative to vaccination determined in one of the aims above.

Blood is collected prior to treatment, and on weeks 2, 4, 6, 8, and 10 of treatment for determination of MRD and for immunological assays. Lymph node size is determined at each recheck visit. A CBC is performed at each recheck to assess numbers of monocytes and neutrophils. At the completion of the study, dogs continue to be followed by telephone follow-up to determine the time of first tumor recurrence (disease-free interval; DFI).

*Preparation of tumor vaccine and LC for MSC depletion.* Autologous tumor vaccines are prepared using lymphoma cells collected from lymph node biopsies obtained from each patient prior to administration of chemotherapy. Single cell suspensions of tumor cells are prepared using gentle enzymatic dissociation. The tumor cells are then fixed overnight in a 1% solution of paraformaldehyde in PBS, which is designed to lightly fix and kill tumor cells, while still preserving surface antigens. Aliquots of fixed tumor cells are stored frozen until used to produce the vaccine. The vaccine is prepared using 1 x 10⁷ tumor cells admixed with 2 ml CLDC adjuvant, using a technique similar to that reported previously to prepare an allogeneic tumor vaccine for dogs with hemangiosarcoma (38). The vaccine is administered intradermally in 2 different sites over the lateral thorax. Vaccination is repeated for a total of 5 immunizations at 2-week intervals. Depletion of MSC is accomplished by i.v. administration of LC, which is prepared as described for treatment of dogs with malignant histiocytosis (34). The LC is administered once every 2 weeks by slow i.v. infusion over 60 minutes, at a dose of 0.5 ml/kg. This dose of LC has been well-tolerated by dogs previously, with transient fever being the most frequent adverse effect in approximately 30% of treated dogs with MH.

*Assessment of vaccine responses*. Vaccine responses is assessed using PBMCs collected prior to treatment and on weeks 2, 4, 6, 8, and 10 of treatment. The PBMCs are thawed and then incubated with PFA-fixed autologous lymphoma cells at 3 different ratios (1:1, 1:10, 1:100) for 96 hours, and proliferation assessed using BrDU incorporation and flow cytometry. In addition, supernatants from the cultures are collected and assayed for determination of IFN-γ concentrations, using a commercial canine IFN-γ ELISA (R & D Systems). A neoantigen (KLH) is incorporated into the vaccine in order to facilitate assessment of vaccine responses, as reported previously (39). Immune responses to KLH are assessed by proliferation and IFN-γ release, using PBMC incubated with 50 ug/ml KLH *in vitro* for 96 hours. In addition, antibody responses to KLH are assessed using a KLH ELISA (39).

*Assessment of molecular remission following chemotherapy and vaccination.* Tumor samples are collected at the beginning of the study to design tumor BCR-specific primer sets for amplification of tumor BCR (40, 41). Blood samples for PCR determination of numbers of circulating lymphoma cells (MRD) are collected at the completion of chemotherapy (immediately prior to first vaccine) and at 2-week intervals during the treatment phase of the study. PBMC are separated and frozen in 3 different aliquots, to be used for MRD calculation and assessment of immune function. Circulating tumor cells are quantitated using quantitative real time PCR (qRT-PCR) and a previously described protocol for quantitation of MRD burden in dogs with B cell lymphoma (37). In that study, which utilized PCR primers designed specifically for an individual patients idiotype Ig, the PCR technique was reported to be sensitive enough to detect circulating tumor cells in each of 7 dogs, even following complete visible tumor remission that was induced using conventional chemotherapy. Moreover, in all 7 studied dogs, the circulating tumor burden increased after the cessation of chemotherapy and the assay was predictive for time to macroscopic tumor recurrence. Thus, the qRT-PCR approach achieves accurate quantitation of the tumor response to vaccination and MSC depletion (i.e., molecular remission). In addition, the between group comparisons should be sufficiently robust to address the primary question of the study (i.e., is combined vaccination/MSC depletion treatment more effective than either alone) without having to include an additional group of dogs with lymphoma treated only with chemotherapy.

Without being bound by theory, the combined treatment with the autologous tumor vaccine and LC yields greater reduction, even significantly greater reduction, in tumor MRD, compared to dogs receiving the tumor vaccine alone or LC treatment alone. Vaccination alone or LC treatment alone also significantly reduces MRD compared to pre-treatment values, but that the combined vaccine/LC treatment generates synergistic antitumor activity. While MRD reduction is the primary endpoint of the study, the immune assays (proliferation, cytokine production, target cell killing) correlate with MRD assays.

### Bibliographic Citations

1. Veelken, H., and F. Osterroth. 2002. Vaccination strategies in the treatment of lymphomas. Oncology 62:187-200.
2. Leitch, H. A., and J. M. Connors. 2005. Vaccine therapy for non-Hodgkin's lymphoma and other B-cell malignancies. Curr Opin Investing Drugs 6:597-604.
3. Park, H. J., and S. S. Neelapu. 2008. Developing idiotype vaccines for lymphoma: from preclinical studies to phase III clinical trials. British journal of haematology 142:179-191.
4. Briones, J. 2008. Therapeutic vaccines for non-Hodgkin B-cell lymphoma. Clin Transl Oncol 10:543-551.
5. Houot, R., and R. Levy. 2009. Vaccines for lymphomas: idiotype vaccines and beyond. Blood reviews 23:137-142.
6. Bendandi, M. 2004. The role of idiotype vaccines in the treatment of human B-cell malignancies. Expert review of vaccines 3:163-170.
7. Gabrilovich, D. I., and S. Nagaraj. 2009. Myeloid-derived suppressor cells as regulators of the immune system. Nat Rev Immunol 9:162-174.
8. Pollard, J. W. 2004. Tumour-educated macrophages promote tumour progression and metastasis. Nat Rev Cancer 4:71-78.
9. Ostrand-Rosenberg, S., and P. Sinha. 2009. Myeloid-derived suppressor cells: linking inflammation and cancer. J Immunol 182:4499-4506.
10. Ostrand-Rosenberg, S., P. Sinha, E. A. Danna, S. Miller, C. Davis, and S. K. Dissanayake. 2004. Antagonists of tumor-specific immunity: tumor-induced immune suppression and host genes that co-opt the anti-tumor immune response. Breast Dis 20:127-135.
11. Kusmartsev, S., and D. I. Gabrilovich. 2006. Role of immature myeloid cells in mechanisms of immune evasion in cancer. Cancer Immunol Immunother 55:237-245.
12. Heithoff, D. M., E. Y. Enioutina, D. Bareyan, R. A. Daynes, and M. J. Mahan. 2008. Conditions that diminish myeloid-derived suppressor cell activities stimulate cross-protective immunity. Infect Immun 76:5191-5199.
13. Nagaraj, S., M. Collazo, C. A. Corzo, J. I. Youn, M. Ortiz, D. Quiceno, and D. I. Gabrilovich. 2009. Regulatory Myeloid Suppressor Cells in Health and Disease. Cancer Res.
14. Almand, B., J. I. Clark, E. Nikitina, J. van Beynen, N. R. English, S. C. Knight, D. P. Carbone, and D. I. Gabrilovich. 2001. Increased production of immature myeloid cells in cancer patients: a mechanism of immunosuppression in cancer. J Immunol 166:678-689.
15. Filipazzi, P., R. Valenti, V. Huber, L. Pilla, P. Canese, M. Iero, C. Castelli, L. Mariani, G. Parmiani, and L. Rivoltini. 2007. Identification of a new subset of myeloid suppressor cells in peripheral blood of melanoma patients with modulation by a granulocyte-macrophage colonystimulation factor-based antitumor vaccine. J Clin Oncol 25:2546-2553.
16. Serafini, P., R. Carbley, K. A. Noonan, G. Tan, V. Bronte, and I. Borrello. 2004. High-dose granulocyte-macrophage colony-stimulating factor-producing vaccines impair the immune response through the recruitment of myeloid suppressor cells. Cancer Res 64:6337-6343.
17. Kusmartsev, S., F. Cheng, B. Yu, Y. Nefedova, E. Sotomayor, R. Lush, and D. Gabrilovich. 2003. All-trans-retinoic acid eliminates immature myeloid cells from tumor-bearing mice and improves the effect of vaccination. Cancer Res 63:4441-4449.
18. Mirza, N., M. Fishman, I. Fricke, M. Dunn, A. M. Neuger, T. J. Frost, R. M. Lush, S. Antonia, and D. I. Gabrilovich. 2006. All-trans-retinoic acid improves differentiation of myeloid cells and immune response in cancer patients. Cancer Res 66:9299-9307.
19. Morse, M. A., J. R. Hall, and J. M. Plate. 2009. Countering tumor-induced immunosuppression during immunotherapy for pancreatic cancer. Expert Opin Biol Ther 9:331-339.
20. De Santo, C., P. Serafini, I. Marigo, L. Dolcetti, M. Bolla, P. Del Soldato, C. Melani, C. Guiducci, M. P. Colombo, M. Iezzi, P. Musiani, P. Zanovello, and V. Bronte. 2005. Nitroaspirin corrects immune dysfunction in tumor-bearing hosts and promotes tumor eradication by cancer vaccination. Proc Natl Acad Sci U S A 102:4185-4190.
21. Sinha, P., V. K. Clements, and S. Ostrand-Rosenberg. 2005. Reduction of myeloid-derived suppressor cells and induction of M1 macrophages facilitate the rejection of established metastatic disease. J Immunol 174:636-645.
22. Claassen, E., N. Kors, and N. van Rooijen. 1987. Immunomodulation with liposomes: the immune response elicited by liposomes with entrapped dichloromethylene-diphosphonate and surface-associated antigen or hapten. Immunology 60:509-515.
23. Claassen, I., N. Van Rooijen, and E. Claassen. 1990. A new method for removal of mononuclear phagocytes from heterogeneous cell populations in vitro, using the liposomemediated macrophage 'suicide' technique. J Immunol Methods 134:153-161.
24. Randolph, G. J., C. Jakubzick, and C. Qu. 2007. Antigen presentation by monocytes and monocyte-derived cells. Curr Opin Immunol.
25. Tacke, F., F. Ginhoux, C. Jakubzick, N. van Rooijen, M. Merad, and G. J. Randolph. 2006. Immature monocytes acquire antigens from other cells in the bone marrow and present them to T cells after maturing in the periphery. J Exp Med 203:583-597.
26. Van Rooijen, N., N. Kors, M. vd Ende, and C. D. Dijkstra. 1990. Depletion and repopulation of macrophages in spleen and liver of rat after intravenous treatment with liposome-encapsulated dichloromethylene diphosphonate. Cell Tissue Res 260:215-222.
27. van Rooijen, N. 1992. Liposome-mediated elimination of macrophages. Res Immunol 143:215-219.
28. Van Rooijen, N., and A. Sanders. 1994. Liposome mediated depletion of macrophages: mechanism of action, preparation of liposomes and applications. J Immunol Methods 174:83-93.
29. Zeisberger, S. M., B. Odermatt, C. Marty, A. H. Zehnder-Fjallman, K. Ballmer-Hofer, and R. A. Schwendener. 2006. Clodronate-liposome-mediated depletion of tumour-associated macrophages: a new and highly effective antiangiogenic therapy approach. Br J Cancer 95:272-281.
30. Condeelis, J., and J. W. Pollard. 2006. Macrophages: obligate partners for tumor cell migration, invasion, and metastasis. Cell 124:263-266.
31. Gazzaniga, S., A. I. Bravo, A. Guglielmotti, N. van Rooijen, F. Maschi, A. Vecchi, A. Mantovani, J. Mordoh, and R. Wainstok. 2007. Targeting tumor-associated macrophages and inhibition of MCP-1 reduce angiogenesis and tumor growth in a human melanoma xenograft. J Invest Dermatol 127:2031-2041.
32. Bosio, C. M., and S. W. Dow. 2005. Francisella tularensis induces aberrant activation of pulmonary dendritic cells. J Immunol 175:6792-6801.
33. Mathes, M., M. Jordan, and S. Dow. 2006. Evaluation of liposomal clodronate in experimental spontaneous autoimmune hemolytic anemia in dogs. Exp Hematol 34:1393-1402.
34. Hafeman, S., C. London, R. Elmslie, and S. Dow. 2009. Evaluation of liposomal clodronate for treatment of malignant histiocytosis in dogs. Cancer Immunol Immurtother.
35. Youn, J. I., S. Nagaraj, M. Collazo, and D. I. Gabrilovich. 2008. Subsets of myeloid-derived suppressor cells in tumor-bearing mice. J Immunol 181:5791-5802.
36. Zaks, K., M. Jordan, A. Guth, K. Sellins, R. Kedl, A. Izzo, C. Bosio, and S. Dow. 2006. Efficient immunization and cross-priming by vaccine adjuvants containing TLR3 or TLR9 agonists complexed to cationic liposomes. J Immunol 176:7335-7345.
37. Yamazaki, J., K. Baba, Y. Goto-Koshino, A. Setoguchi-Mukai, Y. Fujino, K. Ohno, and H. Tsujimoto. 2008. Quantitative assessment of minimal residual disease (MRD) in canine lymphoma by using real-time polymerase chain reaction. Veterinary immunology and immunopathology 126:321-331.
38. U'Ren, L. W., B. J. Biller, R. E. Elmslie, D. H. Thamm, and S. W. Dow. 2007. Evaluation of a novel tumor vaccine in dogs with hemangiosarcoma. Journal of veterinary internal medicine / American College of Veterinary Internal Medicine 21:113-120.
39. Walter, C. U., B. J. Biller, S. E. Lana, A. M. Bachand, and S. W. Dow. 2006. Effects of chemotherapy on immune responses in dogs with cancer. Journal of veterinary internal medicine / American College of Veterinary Internal Medicine 20:342-347.
40. Burnett, R. C., W. Vernau, J. F. Modiano, C. S. Olver, P. F. Moore, and A. C. Avery. 2003. Diagnosis of canine lymphoid neoplasia using clonal rearrangements of antigen receptor genes. Veterinary pathology 40:32-41.
41. Lana, S. E., T. L. Jackson, R. C. Burnett, P. S. Morley, and A. C. Avery. 2006. Utility of polymerase chain reaction for analysis of antigen receptor rearrangement in staging and predicting prognosis in dogs with lymphoma. Journal of veterinary internal medicine / American College of Veterinary Internal Medicine 20:329-334.
42. Avery, A. 2009. Molecular diagnostics of hematologic malignancies. Topics in companion animal medicine 24:144-150.

### Example 7 Clinical Trial of Monocyte/Macrophage Activator L-MTP-PE

Activated monocytes and macrophages eliminate chemotherapy-resistant cancer cells *in vitro,* and therefore agents that activate these effector cells of innate immunity may complement chemotherapy. The minimal peptidoglycan motif muramyl dipeptide (MDP), composed of N-acetylmuramic acid linked to an L-alanine D-isoglutamine dipeptide, is a common membrane component of Gram-negative and Gram-positive bacteria. An important component of complete Freund's adjuvant, MDP activates monocytes and macrophages through the innate immune receptor NALP3. Muramyl tripeptide phosphatidylethanolamine (MTP-PE) is a synthetic conjugation of alanine and dipalmitoylphosphatidylethanolamine to MDP, creating a lipophilic molecule with greater potency, improving cellular uptake and boosting tumoricidal activity. The lipophilic MTP-PE is also more readily incorporated into liposomes for rapid uptake by phagocytic cells. Pharmacokinetics studies in dogs confirmed rapid clearance and a 10-fold reduction in toxicity. Based on promising preclinical studies, clinical trials were conducted in several canine and feline cancers. Following surgical resection, L-MTP-PE was administered at a dose of 2 mg/m2 twice weekly for 8 weeks alone or in combination with chemotherapy (doxorubicin and cyclophosphamide, or cisplatin). When administered immediately after surgery, L-MTP-PE treatment conferred a median survival time of 222 days, significantly longer (p < 0.002) than dogs treated with placebo liposomes (77 days). Non-metastatic dogs treated with L-MTP-PE after cisplatin had a median survival time of 14.4 months, again significantly longer (p < 0.01) than dogs treated with cisplatin and placebo (9.8 months); treatment with L-MT-PE concurrent with cisplatin also improved median survival, but the 1.6 month difference was not significant. Longer disease free survival was also noted in treatment of early-stage melanoma, but had no effect in feline or canine mammary tumors following mastectomy.

Building on the success of these studies in companion animals, a series of exploratory phase I studies were conducted in approximately 150 patients with various advanced cancers (breast, colorectal, lung, melanoma, renal cell carcinoma, stomach and salivary gland cancers as well as sarcoma). These studies determined the L-MTP-PE maximum tolerated dose and optimal biological dose, indicating similar dosing to the canine studies. From 1993-1997, a phase III clinical study assessed the efficacy of L-MTP-PE (mifamurtide) and/or ifosfamide added to the standard regimen of doxorubicin, cisplatin, and high-dose methotrexate in newly diagnosed patients with high-grade osteosarcoma. The trial included 678 patients with non-metastatic resectable osteosarcoma, 332 receiving L-MTP-PE, and 115, and 115 patients with metastatic or unresectable osteosarcoma, with 39 receiving L-MTP-PE. Addition of ifosfamide and three chemotherapeutic drugs did not significantly improve drug free survival (DFS) or overall survival (OS) relative to the standard of care, but addition of L-MTP-PE significantly improved both (DFS p=0.030; OS p=0.039). IDM Pharma Inc submitted an NDA for L-MTP-PE in 2006, but received a nonapprovable letter in 2007 requesting additional data. In March 2009, L-MT-PE (mifamurtide, MEPACT^{®}) was granted a centralized marketing authorization by the European Commission, permitting marketing of the drug in the European Union.

The development pathway of L-MTP-PE exemplifies the way in which studies in human and canine osteosarcoma can proceed in parallel, providing a two-way flow of information that can lead to optimization of drugs for the treatment of osteosarcoma in both species.

### Example 8 Optimization of Electrochemotherapy (ECT)

Some drugs, including cancer chemotherapeutics bleomycin and cisplatin, are highly lipophobic and therefore have poor cellular uptake. Bleomycin is so lipophobic it cannot enter target cells through simple diffusion, requiring relatively slow and inefficient uptake through specific protein receptors resulting in < 0.1% internalized in cultured cells. The high systemic doses required due to poor uptake have caused considerable toxicity to normal tissue, impeding the adoption of bleomycin as an anti-cancer agent despite its therapeutic potential. Short electric pulses that temporarily alter target cell permeability offered a solution to this problem. These pulses appear to induce pores in the cell membrane, improving cellular entry of drugs and plasmids. Electropulsation of cells *in vitro* increased the cytotoxicity of bleomycin several thousand-fold, and increased the cytotoxicity of cisplatin by seventy-fold. The first *in vivo* study of this technique was conducted in 1997 in cats with recurring soft tissue sarcoma after adjuvant radiation therapy. A small cohort of cats received bleomycin followed by square pulses, with prolonged survival in 12 cats relative to 11 untreated controls.

In a subsequent phase I/II study, canine and feline soft tissue sarcoma patients were treated with intralesional bleomycin coupled with biphasic electric pulses, resulting in an overall response rate of 80%, including 40% with long term remissions. This study revealed that canine hemagiopericytomas were particularly responsive to electrochemotherapy (ECT), but also underscored the need for development of customized electrodes adapted to connective tissue. A series of phase II studies were subsequently initiated with the optimized electrodes. Cats with soft tissue sarcoma receiving intraoperative or postoperative bleomycin with electrotherapy had improved average time of recurrence of 12 and 19 months respectively, compared to an average of 4 months with surgery alone. A similar study with canine soft tissue sarcoma patients yielded a median time to recurrence of 730 days and a 95% response rate in dogs treated with bleomycin and electric pulses, with the greatest sensitivity by hemangiopericytomas. A review of over 370 biopsy specimens from ECT trials in a variety of tumors showed a strong correlation between overall survival and necrosis (p < 0.0001) and high rates of apoptosis (p < 0.0001).

The period and frequency of electric pulses were also optimized through multiple trials in companion animals, demonstrating that decreasing the period of pulses from 1 second to 100 milliseconds and increasing the repetition frequency from 1 Hz to 5000 Hz could deliver the necessary 400 V/cm electric field to the tumor with less patient discomfort. Although the first *in vivo* studies were initiated just over a decade ago, ECT is already approved for human use and reimbursed in several EU countries. Clinical studies of ECT in veterinary patients began shortly after the first human oncology trials, and the approach is used extensively in several European countries and Brazil for cats, dogs, and horses with a wide variety of cutaneous and subcutaneous tumors. Optimization of the technique has progressed in parallel in human and veterinary clinical trials, exemplifying how similarities between tumors in humans and companion animals and communication between oncologists working in both fields can accelerate development of new therapeutic modalities.

### Example 9 Treatment of Hemangiosarcomas

Liposome encapsulated muramyl tripeptide phosphatidylethanolamine (L-MTP-PE) proved successful in randomized clinical studies of canine osteosarcoma (above), and therefore this therapeutic strategy was extended to hemangiosarcoma. Thirty-two dogs with HSA and no evident metastases were treated with splenectomy and doxorubicin + cyclophosphamide along with L-MTP-PE or placebo. Dogs that received L-MTP-PE had significantly improved disease-free survival (p=0.037) and overall survival (p=0.029), with better responses by dogs in clinical stage I than in clinical stage II. Bioassay showed significant elevation of serum tumor necrosis factor and interleukin-6, important immune cytokines. These studies suggest a novel therapeutic approach for this unmet medical need in dogs. Furthermore, studies of canine HSA may inform anti-metastatic strategies for treatment of companion animals and humans.

### Example 10 Plasmid DNA Stimulation of Innate and Adaptive Immunity

T cells activated by bacterial superantigens develop strong cytolytic activity and mediate tumor regression when adoptively transferred. Twenty-six dogs with spontaneous malignant melanoma were treated with plasmid DNA encoding the bacterial superantigen staphylococcal enterotoxin B and either GM-CSF or IL-2 to test the effect of DNA vaccination on tumor regression. The overall response rate (complete and partial remission) for all dogs was 46%, and was highest in smaller tumors. Histological examination revealed CD4+ and CD8+ T cell infiltrates in the tumors, and demonstrated that tumor regression was correlated with high levels of circulating cytotoxic T lymphocytes. In this study, the plasmid DNA was complexed with cationic lipids to compact the plasmid for greater stability. Subsequent studies revealed that the combination of cationic lipid and bacterial DNA effectively stimulated innate immunity and provoked a strong cytokine response even in the absence of encoded genes.

### Example 11 Development of Antiangiogenic Thrombospondin-1 Peptide Mimetics

This example shows how spontaneous tumors in companion animals can play a key role in bridging therapeutic development from mouse models to human clinical trials. As tumors grow they must induce localized angiogenesis to develop an adequate blood supply supporting further growth. Therefore, blocking angiogenesis is a goal of many cancer therapy efforts. Thrombospondin-1 (TSP-1) is a pleiotropic natural angiogenesis inhibitor, blocking many aspects of endothelial cell activation. Modified nonapeptides based on the angiogenic domain of TSP-1, ABT-526 and ABT-510, share this antagonist activity in a more practical size for drug development. Initial efficacy studies in syngeneic and xenograft mouse models showed that ABT-526 and ABT-510 both slow tumor growth. However, inhibition of angiogenesis is unlikely to rapidly destroy tumors, so establishing the dose for human clinical trial based on a rapidly progressing mouse cancer model was not considered optimal. To better define safety and efficacy, the two TSP-1 peptide mimetics were tested in an open-label nonclinical trial of spontaneous canine tumors. A prospective open-label trial was conducted on 242 dogs with a variety of cancers including NHL, soft tissue sarcoma, mammary adenocarcinoma, head and neck carcinoma, and many other primary and metastatic tumors (115). Pharmacokinetic studies were conducted in a laboratory colony of beagle dogs, providing a bridge between mouse and outbred companion animal studies and establishing initial dose parameters. No dose-limiting toxicities were observed in any dogs in the study. Objective regression (> 50% reduction of tumor size) of measureable lesions were noted in 19 of 180 evaluable dogs and significant disease stabilization occurred in 23 dogs. Most of these responses occurred after 60 days of treatment with the TSP-1 mimetic, confirming the selection of spontaneous tumors in dogs as the appropriate model to optimize dosing and confirm efficacy. This study indicated that NHL was one of the more responsive classes of tumor and that ABT-526 was more active than ABT-510. Based on these results, a controlled double-blinded trial of ABT-526 was conducted on 94 pet dogs with naturally occurring first-relapse NHL. This study was designed to provide additional definition of optimal biological dose and schedule, identify predictive biomarkers of activity, and to test efficacy in combination with chemotherapy. Dogs received lomustine (CeeNu®, Bristol Myers Squibb) and placebo or ABT-526. In this controlled clinical trial ABT-526 did not increase the number of cases responding to chemotherapy, but modestly enhanced the duration of response. ABT-510 testing was advanced into a series of human phase I and phase II clinical trials. A phase I safety, pharmacokinetic and pharmacodynamic study of ABT-510 in 39 human patients with a range of advanced cancers demonstrated a favorable toxicity profile and caused a decrease in basic fibroblast growth factor, a marker of angiogenesis, and stable disease in 6 patients for at least 6 months.

### Example 12 Reduction of Doxil Adverse Effects

Doxorubicin is an anthracycline antibiotic that intercalates into DNA blocking replication, and is used in the treatment of a wide range of cancers including hematological malignancies such as NHL and in soft tissue sarcomas. Doxil, a peglylated liposome containing doxorubicin, has prolonged circulation and enhanced anti-tumor efficacy with less cardiotoxicity. However, unlike free doxorubicin, Doxil induces a painful skin reaction called palmar-plantar erythrodysesthesia (PPES), sometimes called hand-foot disease. Like humans, dogs are also susceptible to development of PPES following prolonged Doxil therapy. Anecdotal evidence suggested that oral vitamin B6 (pyridoxine) could alleviate or eliminate PPES. To test this, a randomized double-blind study of daily Doxil chemotherapy in combination with oral pyridoxine or placebo was conducted in 41 dogs with NHL (118). No difference was observed in remission rates between treatment groups, but the relative risk of developing PPES was 4.2 times greater in the placebo group. Although pyridoxine did not completely prevent or reverse PPES, it delayed and lessened the symptoms. This exploratory trial in dogs provided the rationale for more extensive testing of this strategy in human patients.

## Claims

1. A method for identifying a combination of anti-cancer agents with synergistic effects comprising (1) monitoring a companion animal with a spontaneously occurring cancer and that has been admistered with two or more anti-cancer agents for a biological and/or physiological effect; and (2) identifying a combination of anti-cancer agents with synergistic effects when the biological and/or physiological effects are synergistic, wherein the anti-cancer agents comprise bisphosphonates and cationic CpG.

2. The method of claim 1 wherein the agents are clodronate and cationic CpG.

3. The method of any one of claims 1 or 2 wherein the companion animal is a dog.

4. The method of claim 3 wherein the companion animal is a purebred dog.

5. The method of claim 3 wherein the companion animal is a mongrel dog.

6. The method of claim 3 wherein the dog has a homogeneous genetic background.

7. The method of claim 3 wherein the dog has a heterogeneous genetic background.

8. The method of any one of claims 1 or 2 wherein the companion animal is a cat.

## Patentansprüche

1. Verfahren zur Identifizierung einer Kombination von Antikrebsmitteln mit synergistischen Wirkungen, umfassend:
(1) Überwachen eines Begleittiers mit spontan auftretendem Krebs, dem zwei oder mehr Antikrebsmittel verabreicht wurden, auf eine biologische und/oder physiologische Wirkung und
(2) Identifizieren einer Kombination von Antikrebsmitteln mit synergistischen Wirkungen, wenn die biologischen und/oder physiologischen Wirkungen synergistisch sind, wobei die Antikrebsmittel Bisphosphonate und kationisches CpG umfassen.

2. Verfahren nach Anspruch 1, wobei die Mittel Clodronat und kationisches CpG sind.

3. Verfahren nach einem der Ansprüche 1 oder 2, wobei das Begleittier ein Hund ist.

4. Verfahren nach Anspruch 3, wobei das Begleittier ein reinrassiger Hund ist.

5. Verfahren nach Anspruch 3, wobei das Begleittier ein Mischlingshund ist.

6. Verfahren nach Anspruch 3, wobei der Hund einen homogenen genetischen Hintergrund hat.

7. Verfahren nach Anspruch 3, wobei der Hund einen heterogenen genetischen Hintergrund hat.

8. Verfahren nach einem der Ansprüche 1 oder 2, wobei das Begleittier eine Katze ist.

## Revendications

1. Procédé visant à identifier une combinaison d'agents anticancéreux présentant des effets de synergie, comportant les étapes suivantes :
1) surveiller, chez un animal familier atteint d'un cancer apparu spontanément, auquel on a administré deux agents anticancéreux ou plus, un effet biologique et/ou physiologique,
2) et identifier une combinaison d'agents anticancéreux présentant des effets de synergie, quand les effets biologiques et/ou physiologiques sont synergiques,
étant entendu que les agents anticancéreux comprennent des bis-phos-phonates et des CpG cationiques.

2. Procédé conforme à la revendication 1, dans lequel les agents sont le clodronate et un CpG cationique.

3. Procédé conforme à l'une des revendications 1 et 2, dans lequel l'animal familier est un chien.

4. Procédé conforme à la revendication 3, dans lequel l'animal familier est un chien de race.

5. Procédé conforme à la revendication 3, dans lequel l'animal familier est un chien bâtard.

6. Procédé conforme à la revendication 3, dans lequel le chien présente un arrière-plan génétique homogène.

7. Procédé conforme à la revendication 3, dans lequel le chien présente un arrière-plan génétique hétérogène.

8. Procédé conforme à l'une des revendications 1 et 2, dans lequel l'animal familier est un chat.
